# EUROPÄISCHE PATENTSCHRIFT

(11) **EP 1 620 155 B1**
(45) Veröffentlichungstag und Bekanntmachung des Hinweises auf die Patenterteilung: **23.07.2008**
(21) Anmeldenummer: 04731343.2
(22) Anmeldetag: 06.05.2004
(51) Int. Cl.: A61M 16/20, A61M 16/00, A61M 39/22, A61M 39/26

(54) **VERNEBLERANSCHLUSSVORRICHTUNG FÜR BEATMUNGSGERÄTE ODER DERGLEICHEN**
NEBULIZER-CONNECTING DEVICE FOR RESPIRATORS OR SIMILAR
ENSEMBLE RACCORD DE NEBULISEUR POUR APPAREILS RESPIRATOIRES OU ANALOGUES

(30) Priorität: 06.05.2003 DE 10320143
(43) Veröffentlichungstag der Anmeldung: 01.02.2006
(73) Patentinhaber: PARI GmbH Spezialisten für effektive Inhalation, 82319 Starnberg (DE)
(72) Erfinder: BOEHM, Andreas, 86934 Reichling (DE); KUMMER, Frank, 80689 München (DE); MORNHINWEG, Markus, 86911 Diessen (DE)
(74) Vertreter: HOFFMANN EITLE
(86) Internationale Anmeldenummer: PCT/EP2004/004842
(87) Internationale Veröffentlichungsnummer: WO 2004/098689

(56) Entgegenhaltungen:
- EP-A- 0 576 380
- WO-A-02/089887
- DE-C- 19 639 870
- FR-A- 1 449 918
- FR-A- 1 552 128
- FR-A- 2 267 801
- FR-A- 2 804 609
- US-A- 3 279 487
- US-A- 4 077 404
- US-A- 4 314 586
- US-A- 4 951 661
- US-A1- 2002 020 409

## Beschreibung

Die vorliegende Erfindung betrifft eine Vernebleranschlussvorrichtung für Beatmungsgeräte und allgemein für Geräte, bei denen einem Benutzer Atemluft über eine Zuführleitung, beispielsweise eine Schlauchleitung, zugeführt wird.

Bei Beatmungsgeräten wird einem zu beatmenden Patienten Atemluft über eine Schlauchleitung zugeführt. Um der Atemluft ein Aerosol zusetzen zu können, muss ein Vernebler an die Schlauchleitung derart angeschlossen werden, dass das von dem Vernebler erzeugte Aerosol in die Atemluft gelangt, die dem Patienten zugeführt wird. Wie aus US 4 951 661 oder WO 02/089887 bekannt ist, kann ein Vernebler mit Hilfe eines T-Stücks, das in die Schlauchleitung eingesetzt wird, an die Atemluftleitung angeschlossen werden, so dass das Aerosol durch das Rohrstück strömt, das senkrecht in das die Atemluft führende Rohrstück einmündet. Auf diese Weise gelangt das erzeugte Aerosol in den Atemluftstrom und wird über die Zuführschlauchleitung zusammen mit der Beatmungsluft dem Patienten zugeführt.

Der Vernebler kann aber in der klinischen Praxis schon aus Kostengründen nicht dauerhaft angeschlossen bleiben, sondern muss nach abgeschlossener Aerosoltherapie wieder aus der Beatmungsleitung entfernt werden. Das Einsetzen und Entfernen des T-Stücks mit dem daran angeschlossenen Vernebler ist problematisch, da dabei die Atemluftzuführung durch die Zuführleitung vom Beatmungsgerät zum Patienten unterbrochen werden muss. Auch wenn versucht wird, die Unterbrechung nur sehr kurz zuhalten, ist ein Einsetzen und Entfernen auf diese Weise nicht akzeptabel. Selbst ohne Trennung der Beatmungsschlauchleitung führt aber auch das Anbringen und Entfernen des Verneblers von dem Anschlussrohrstück zu Problemen, da kurzzeitig das T-Stück an dem senkrecht verlaufenden Anschlussrohrstück offen ist, wodurch die Beatmung des Patienten beeinträchtigt wird.

Überdies ist auf diese Weise nicht sichergestellt, dass das Anschlussrohrstück nach dem Entfernen des Verneblers wieder, beispielsweise mit einem Stopfen, verschlossen wird. Einige dieser Probleme sind in US 4 951 661 oder WO 02/089887 auch angesprochen.

Aus US 4 951 661 ist ein T-Stück mit einem Ventil bekannt, das den T-Stutzen verschließt, wenn kein Vernebler angeschlossen ist ; in der Verschlussstellung wird das Ventilelement aufgrund der Wirkung einer Feder gehalten.

Durch das Anschließen eines Verneblers an den T-Stutzen wird das Ventilelement gegen die Federwirkung angehoben.

Die aus US 4 951 661 bekannte Gestaltung des T-Stücks ist in mehrfacher Hinsicht ungünstig. Die Feder des Ventilelement ist im Strömungsweg des Aerosols angeordnet und behindert dadurch die Zuführung des Aerosols. Außerdem verunreinigt die Feder und ist schlecht zu reinigen. Das Anheben des Ventilelements erfolgt über einen Stempel, der sich ebenfalls im Strömungsweg des Aerosol befindet und die Strömung stört. Überdies ist das angehobene Ventilelement in der Atemluftströmung angeordnet und sorgt auch hier für eine Behinderung der Strömung.

Um einige Fehler der Konstruktion aus US 4 951 661 zu beseitigen, wird in WO 02/089887 vorgeschlagen, die Feder für das Ventilelement nicht mittig im T-Stutzen um den Stempelschaft anzuordnen, sondern in einem an der Wand des T-Stutzens umlaufend angeordneten Gehäuse. Dadurch wird erreicht, dass die Feder in einem geschlossenen Raum angeordnet ist und so nicht verunreinigt werden kann.

Jedoch ist nach wie vor die Konstruktion aus WO 02/089887 nachteilig, da auch hier der Stempel, der Stempelschaft und das Ventilelement strömungsbehindernd angeordnet sind, was zu Aerosolverlusten führt, da sich das Aerosol an den zahlreichen im Strömungsweg angeordneten Oberflächen leicht abscheidet. Durch das Federgehäuse ist der T-Stutzen verengt. Überdies befindet sich im angehobenen Zustand das Ventilelement im Strömungsweg der Atemluft.

Zu beiden Konstruktionen ist anzumerken, dass durch die Feder, deren Rückstellkraft unverzichtbar ist, um ein Offenstehen des T-Stücks nach dem Abnehmen des Verneblers zu vermeiden, beide Gestaltungen sehr aufwändig sind und Probleme bei der Reinigung und Sterilisation bereiten.

Das von der Erfindung zu lösende technische Problem besteht vor diesem Hintergrund darin, eine Vernebleranschlussvorrichtung für Beatmungsgeräte oder dergleichen anzugeben, die ein einfaches Anschließen und Entfernen eines Verneblers ermöglicht, so dass der Atemluft ein Aerosol zugeführt werden kann, bei der aber sichergestellt ist, dass beim Anschließen und Entfernen des Verneblers keine Unterbrechung und keine Beeinträchtigung der Atemluftzuführung erfolgt, die einfach aufgebaut ist und bei der kein strömungsbehindernder Einfluss ausgeübt wird.

Gelöst wird dieses Problem durch eine Vernebleranschlussvorrichtung mit den in Anspruch 1 angegebenen Merkmalen. Vorteilhafte Ausgestaltungen ergeben sich aus den Unteransprüchen.

Erfindungsgemäß wird die Verschlusseinrichtung, die an der Anschlusseinrichtung für den Vernebler an der Atemluftführungseinrichtung angeordnet ist, beim Anschließen und Entfernen des Verneblers geöffnet bzw. geschlossen, so dass die Vorgänge eng miteinander verbunden sind. Dadurch wird schon durch die erfindungsgemäße Anordnung gewährleistet, dass ein Anschließen ohne Öffnen und ein Entfernen ohne Verschließen praktisch ausgeschlossen ist.

Vorteilhaft wirkt der Vernebler oder ein Teil davon, beispielsweise ein Aerosolabgabestutzen auf die Verschlusseinrichtung derart ein, dass ein Öffnen bzw. Schließen des Strömungswegs für das Aerosol erfolgt. Dadurch wird erreicht, dass ohne weitere Hilfsmittel beim Anschließen des Verneblers die Verschlusseinrichtung betätigt werden kann.

Die Erfindung ist nicht nur bei Beatmungsgeräten einsetzbar, bei denen eine Unterbrechung oder Beeinträchtigung der Beatmung zu lebensbedrohenden Situationen führen kann. Die Anschlussvorrichtung gemäß der Erfindung kann auch bei anderen medizintechnischen Geräten eingesetzt werden, beispielsweise Atmungstherapiegeräten oder Lungenfunktionsmessgeräten, wenn der Atemluft während eines Therapie- oder Messvorgangs ein Aerosol zugesetzt und dazu ein Vernebler an eine Zuführleitung für die Atemluft angeschlossen werden soll.

Die Erfindung wird im folgenden anhand von Ausführungsbeispielen unter Bezugnahme auf die Zeichnungen genauer erläutert. Dazu zeigen
Fig. 1 bis 3 ein erstes Ausführungsbeispiel der Erfindung;
Fig. 4 bis 6 ein zweites Ausführungsbeispiel der Erfindung;
Fig. 7 bis 9 ein drittes Ausführungsbeispiel der Erfindung;
Fig. 10 bis 13 ein viertes Ausführungsbeispiel der Erfindung;
Fig. 14 bis 17 ein fünftes Ausführungsbeispiel der Erfindung;
Fig. 18 und 19 ein sechstes Ausführungsbeispiel der Erfindung; und
Fig. 20 bis 22 ein siebtes Ausführungsbeispiel der Erfindung.

Bei der folgenden Darstellung der Ausführungsbeispiele wird die Anwendung der erfindungsgemäßen Vernebleranschlussvorrichtung an einem Beatmungsgerät erläutert. Eine Beschränkung auf diesen Anwendungsfall ist damit aber nicht verbunden oder beabsichtigt, auch wenn die Anwendung der erfindungsgemäßen Vernebleranschlussvorrichtung in der Beatmungssituation eines Beatmungsgeräts besondere Vorteile mit sich bringt, wie sich aus der Erläuterung der Ausführungsbeispiele ergeben wird.

### 1. Ausführungsbeispiel

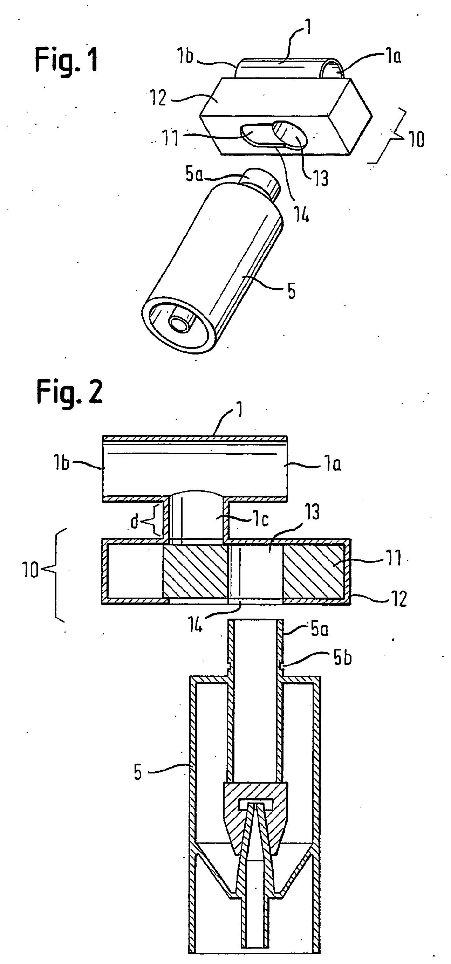

Die Figuren 1 bis 3 zeigen ein erstes Ausführungsbeispiel einer erfindungsgemäßen Vernebleranschlussvorrichtung für Beatmungsgeräte oder dergleichen.

Figur 1 zeigt eine perspektivische Ansicht des ersten Ausführungsbeispiels, aus der sich bereits ein allen Ausführungsbeispielen gemeinsamer Aspekt ergibt. Denn alle Ausführungsbeispiele der erfindungsgemäßen Vernebleranschlussvorrichtung besitzen eine Atemluftführungseinrichtung 1, beispielsweise das in Figur 1 gezeigte Rohrstück 1, das in eine Atemluftleitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

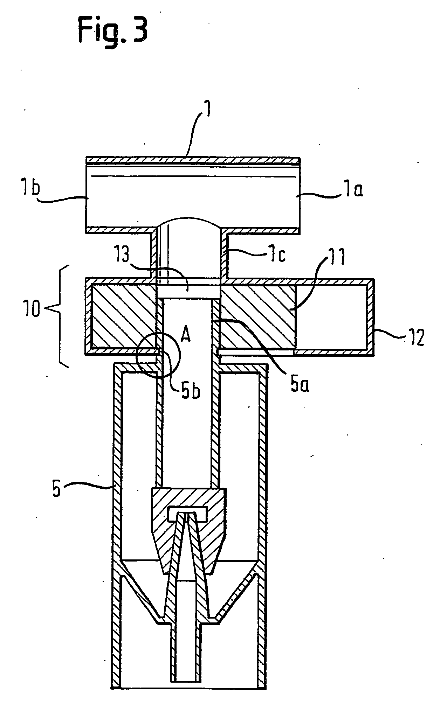

Wie den Figuren 2 und 3 erkennbar, ist an einer dritten Anschlusseinrichtung 1c, beispielsweise dem in den Figuren 2 und 3 dargestellten Rohrstück 1c, das in die Atemluftführungseinrichtung 1 einmündet, erfindungsgemäß eine Verschlusseinrichtung 10 angeordnet. Die Verschlusseinrichtung 10 des ersten Ausführungsbeispiels gemäß der Erfindung umfasst ein Verschlussorgan 11, das in einem Verschlussorgangehäuse 12 angeordnet ist. Figur 2 zeigt das Verschlussorgan 11 in der den Strömungsweg der dritten Anschlusseinrichtung 1c verschließenden Stellung. Wie sich aus Figur 2 ergibt, ist ein Teil des Verschlussorgans 11 vor dem Anschlussstutzen 1c angeordnet, so dass durch den Anschlussstutzen 1c weder Beatmungsluft in die Umgebung noch Umgebungsluft in die Beatmungsluft gelangen kann. Das Verschlussorgan 11 umfasst ein Verschlussorganöffnung 13, in die ein Abgabestutzen 5a eines Verneblers 5 eingeführt werden kann. Dazu besitzt das Verschlussorgangehäuse 12 eine Verschlussorgangehäuseöffnung 14, die so geformt ist, dass ein Abgabestutzen 5a eines Verneblers 5 durch die Verschlussorgangehäuseöffnung 14 hindurch in die Verschlussorganöffnung 13 eingeführt und der Abgabestutzen 5a derart verschoben werden kann, dass das Verschlussorgan 11 in dem Verschlussorgangehäuse 12 verschoben wird. Die geradlinig Bewegung wird vorgegeben durch die quaderartige Gestaltung sowohl des Verschlussorgans 11 als auch des Verschlussorgangehäuses 12, die in Figur 1 erkennbar ist. Wie in Figur 1 auch ersichtlich, ist die Verschlussorgangehäuseöffnung 14 ein Langloch, so dass ein zylindrischer Abgabestutzen 5a eines Verneblers 5 eingeführt und in der Verschlussorgangehäuseöffnung 14 geradlinig verschoben werden kann. Wie sich aus Figur 3 ergibt, ist in der verschobenen Stellung die Verschlussorganöffnung 13 dem Anschlussstutzen 1c gegenüberliegend angeordnet, so dass der Abgabestutzen 5a des Verneblers 5 mit dem Anschlussstutzen 1c ausgerichtet ist. Ein von dem Vernebler erzeugtes Aerosol gelangt so auf ungestörtem Weg aus dem Vernebler 5 durch den Abgabestutzen 5a und den Anschlussstutzen 1c in die Atemluftführungseinrichtung 1 der erfindungsgemäßen Vernebleranschlussvorrichtung.

Beim Entfernen des Verneblers 5 wird zunächst der Vernebler 5 mit dem Abgabestutzen 5a in die in Figur 2 gezeigte Stellung verschoben, wodurch das Verschlussorgan 11 die dritte Anschlusseinrichtung 1c verschließt, wie in Figur 2 gezeigt ist. Dann wird der Abgabestutzen 5a des Verneblers 5 aus der Verschlussorganöffnung 13 herausgezogen.

Erfindungsgemäß ist die dritte Anschlusseinrichtung 1c verschlossen, so dass weder Beatmungsluft in die Umgebung noch Umgebungsluft in die Beatmungsluft gelangen kann.

Um zu vermeiden, dass bei der in Figur 3 gezeigten Stellung der Abgabestutzen 5a des Verneblers 5 unbeabsichtigt aus der Verschlussorganöffnung 13 herausgezogen wird, ist die Verschlussorgangehäuseöffnung 14 in einem dem Anschlussstutzen 1c gegenüberliegenden Bereich vorzugsweise enger ausgeführt als in einem Bereich, der mit der Position der Verschlussorganöffnung 13 in der in Figur 2 gezeigten Stellung ausgerichtet ist. Der Außendurchmesser des Abgabestutzen 5a ist größer als die Breite der Verschlussorgangehäuseöffnung 14 in dem verengten Bereich. Damit der Abgabestutzen 5a des Verneblers 5 in den engeren Bereich der Verschlussorgangehäuseöffnung 14 verschoben werden kann, besitzt der Abgabestutzen 5a eine Einkerbung 5b, die die Außenabmessung des Abgabestutzens 5a ausreichend verringert, um in den engeren Bereich der Verschlussorgangehäuseöffnung 14 ein-geschoben werden zu können. In Figur 3 ist an der Stelle A erkennbar, wie das Verschlussorgangehäuse 12 in die Einkerbung 5b des Abgabestutzens 5a eingreift, wodurch verhindert wird, dass der Abgabestutzen 5a des Verneblers 5 in der in Figur 3 gezeigten Stellung aus der Verschlussorganöffnung 13 herausgezogen wird. Da sich der enge Bereich der Verschlussorgangehäuseöffnung 14 so weit erstreckt, dass der Eingriff des Verschlussorgangehäuses 12 in die Einkerbung 5b des Abgabestutzens 5a erst aufgehoben ist, wenn das Verschlussorgan 11 die in Figur 2 gezeigte Position erreicht hat, ist sichergestellt, dass auch in einer Zwischenstellung zwischen der Verneblungsstellung des Verschlussorgans 11 gemäß Figur 3 und der Verschließstellung des Verschlussorgans 11 gemäß Figur 2 der Abgabestutzen 5a des Verneblers aus der Verschlussorganöffnung 13 nicht herausgezogen werden kann.

Wie in Figur 2 erkennbar, ist der Abstand d zwischen der Verschlusseinrichtung 10 und der Atemluftführungseinrichtung 1 der erfindungsgemäßen Vernebleranschlussvorrichtung nahezu beliebig verkürzbar. Wenn der Abstand d minimiert wird, d.h. wenn die Verschlusseinrichtung 10 unmittelbar neben der Atemluftführungseinrichtung 1 angeordnet ist, wird erreicht, dass im geschlossenen Zustand praktisch kein Totvolumen entsteht und die Beatmungsluft nicht beeinträchtigt wird, die durch die Atemluftführungseinrichtung 1 strömt. Auch für das Aerosol, das über den Abgabestutzen 5a aus dem Vernebler in die Beatmungsluft strömt, ist der Strömungsweg minimiert, wenn der Abstand d so weit wie möglich verkürzt wird.

### 2. Ausführungsbeispiel

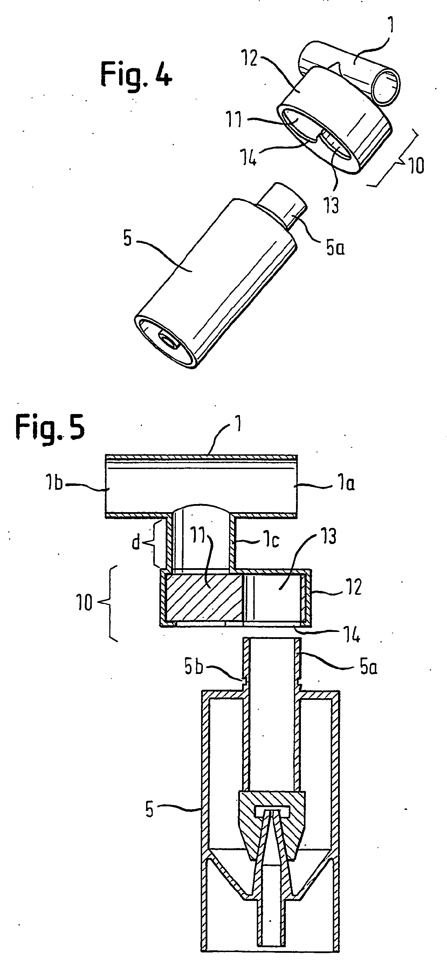

Die Figuren 4 bis 6 zeigen ein zweites Ausführungsbeispiel einer erfindungsgemäßen Vernebleranschlussvorrichtung für Beatmungsgeräte oder dergleichen.

Aus den Figuren 4 bis 6 ergibt sich zunächst, dass auch das zweite Ausführungsbeispiel, wie die anderen Ausführungsbeispiele, eine Atemluftführungseinrichtung 1, beispielsweise das in Figur 4 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

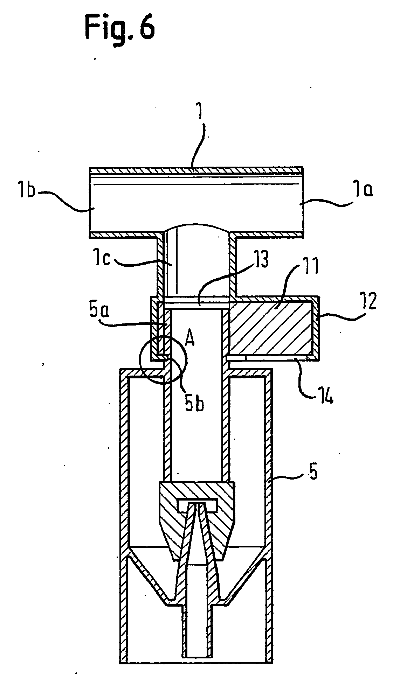

Wie den Figuren 5 und 6 erkennbar, ist an einer dritten Anschlusseinrichtung 1c, beispielsweise dem in den Figuren 5 und 6 erkennbaren Rohrstück, das in die Atemluftführungseinrichtung 1 einmündet, erfindungsgemäß eine Verschlusseinrichtung 10 angeordnet. Die Verschlusseinrichtung 10 des zweiten Ausführungsbeispiels gemäß der Erfindung umfasst ein Verschlussorgan 11, das in einem Verschlussorgangehäuse 12 angeordnet ist. Figur 5 zeigt das Verschlussorgan 11 in der den Strömungsweg der dritten Anschlusseinrichtung 1c verschließenden Stellung. Wie sich aus Figur 5 ergibt, ist ein Teil des Verschlussorgans 11 vor dem Anschlussstutzen 1c angeordnet, so dass durch den Anschlussstutzen 1c weder Beatmungsluft in die Umgebung noch Umgebungsluft in die Beatmungsluft gelangen kann. Das Verschlussorgan 11 umfasst ein Verschlussorganöffnung 13, in die ein Abgabestutzen 5a eines Verneblers 5 eingeführt werden kann. Dazu besitzt das Verschlussorgangehäuse 12 eine Verschlussorgangehäuseöffnung 14, die so geformt ist, dass der Abgabestutzen 5a des Verneblers 5 durch die Verschlussorgangehäuseöffnung 14 hindurch in die Verschlussorganöffnung 13 eingeführt und der Abgabestutzen 5a derart entlang eines Kreisabschnitts bewegt werden kann, dass das Verschlussorgan 11 in dem Verschlussorgangehäuse 12 verdreht wird. Die Bewegung entlang einer Kreisbahn wird vorgegeben durch die zylindrische Gestaltung sowohl des Verschlussorgans 11 als auch des Verschlussorgangehäuses 12, die in Figur 4 erkennbar ist. Wie aus Figur 4 ferner ersichtlich, ist die Verschlussorgangehäuseöffnung 14 ein entlang eines Kreisabschnitts verlaufendes Langloch, so dass ein zylindrischer Abgabestutzen 5a eines Verneblers 5 eingeführt und in der Verschlussorgangehäuseöffnung 14 entlang einer Kreisbahn bewegt werden kann. Wie sich aus Figur 6 ergibt, ist in der verdrehten Stellung die Verschlussorganöffnung 13 dem Anschlussstutzen 1c gegenüberliegend angeordnet, so dass der Abgabestutzen 5a des Verneblers 5 mit dem Anschlussstutzen 1c ausgerichtet ist. Ein von dem Vernebler erzeugte Aerosol gelangt so auf ungestörtem Weg aus dem Vernebler 5 durch den Abgabestutzen 5a und den Anschlussstutzen 1c in die Atemluftführungseinrichtung 1 der erfindungsgemäßen Vernebleranschlussvorrichtung.

Beim Entfernen des Verneblers 5 wird zunächst der Vernebler 5 mit dem Abgabestutzen 5a in die in Figur 5 gezeigte Stellung bewegt, wodurch das Verschlussorgan 11 die dritte Anschlusseinrichtung 1c verschließt, wie in Figur 5 gezeigt ist. Dann wird der Abgabestutzen 5a des Verneblers 5 aus der Verschlussorganöffnung 13 herausgezogen. Erfindungsgemäß ist nun die dritte Anschlusseinrichtung 1c verschlossen, so dass weder Beatmungsluft in die Umgebung noch Umgebungsluft in die Beatmungsluft gelangen kann.

Um zu vermeiden, dass bei der in Figur 6 gezeigten Stellung der Abgabestutzen 5a des Verneblers 5 unbeabsichtigt aus der Verschlussorganöffnung 13 herausgezogen wird, ist die Verschlussorgangehäuseöffnung 14 in einem dem Anschlussstutzen 1c gegenüberliegenden Bereich enger ausgeführt, als in einem Bereich, der mit der Position der Verschlussorganöffnung 13 in der in Figur 5 gezeigten Stellung ausgerichtet ist. Der Außendurchmesser des Abgabestutzen 5a ist größer als die Breite der Verschlussorgangehäuseöffnung 14 in dem verengten Bereich. Damit der Abgabestutzen 5a des Verneblers 5 in den engeren Bereich der Verschlussorgangehäuseöffnung 14 bewegt werden kann, besitzt der Abgabestutzen 5a eine Einkerbung 5b, die die Außenabmessung des Abgabestutzens 5a ausreichend verringert, um in den engeren Bereich der Verschlussorgangehäuseöffnung 14 eingeschoben werden zu können. In Figur 6 ist an der Stelle A erkennbar, wie das Verschlussorgangehäuse 12 in die Einkerbung 5b des Abgabestutzens 5a eingreift, wodurch verhindert wird, dass der Abgabestutzen 5a des Verneblers 5 in der in Figur 6 gezeigten Stellung aus der Verschlussorganöffnung 13 herausgezogen wird. Da sich der enge Bereich der Verschlussorgangehäuseöffnung 14 so weit erstreckt, dass der Eingriff des Verschlussorgangehäuses 12 in die Einkerbung 5b des Abgabestutzens 5a erst aufgehoben ist, wenn das Verschlussorgan 11 die in Figur 5 gezeigte Position erreicht hat, ist sichergestellt, dass auch in einer Zwischenstellung zwischen der Verneblungsstellung des Verschlussorgans 11 gemäß Figur 6 und der Verschließstellung des Verschlussorgans 11 gemäß Figur 5 der Abgabestutzen 5a des Verneblers 5 aus der Verschlussorganöffnung 13 nicht herausgezogen werden kann.

Wie aus Figur 5 entnehmbar, ist der Abstand d zwischen der Verschlusseinrichtung 10 und der Atemluftführungseinrichtung 1 der erfindungsgemäßen Vernebleranschlussvorrichtung nahezu beliebig verkürzbar. Wenn der Abstand d minimiert wird, d.h. wenn die Verschlusseinrichtung 10 unmittelbar neben der Atemluftführungseinrichtung 1 angeordnet ist, wird erreicht, dass im geschlossenen Zustand kein Totvolumen entsteht und dass die Beatmungsluft nicht beeinträchtigt wird, die durch die Atemluftführungseinrichtung 1 strömt. Auch für das Aerosol, das über den Abgabestutzen 5a aus dem Vernebler in die Beatmungsluft strömt, ist der Strömungsweg minimiert, wenn der Abstand d so weit wie möglich verkürzt wird.

### 3. Ausführungsbeispiel

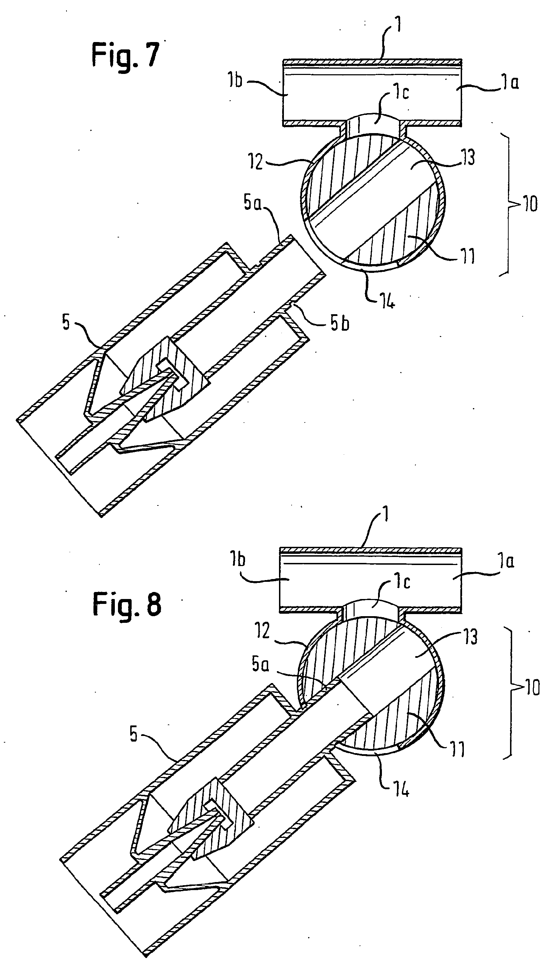

Die Figuren 7 bis 9 zeigen ein drittes Ausführungsbeispiel einer erfindungsgemäßen Vernebleranschlussvorrichtung für Beatmungsgeräte oder dergleichen.

Aus den Figuren 7 bis 9 ergibt sich zunächst, dass auch das dritte Ausführungsbeispiel, wie die anderen Ausführungsbeispiele, eine Atemluftführungseinrichtung 1, beispielsweise das in Figur 7 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

Wie den Figuren 7 bis 9 erkennbar, ist an einer dritten Anschlusseinrichtung 1c, beispielsweise dem in den Figuren 7 bis 9 erkennbaren Rohrstück, das in die Atemluftführungseinrichtung 1 einmündet, erfindungsgemäß eine Verschlusseinrichtung 10 angeordnet. Die Verschlusseinrichtung 10 des dritten Ausführungsbeispiels gemäß der Erfindung umfasst ein Verschlussorgan 11, das in einem Verschlussorgangehäuse 12 angeordnet ist. Die Figuren 7 und 8 zeigen das Verschlussorgan 11 in der den Strömungsweg der dritten Anschlusseinrichtung 1c verschließenden Stellung. Wie sich aus den Figuren 7 und 8 ergibt, ist ein Teil des Verschlussorgans 11 vor dem Anschlussstutzen 1c angeordnet, so dass durch den Anschlussstutzen 1c weder Beatmungsluft in die Umgebung noch Umgebungsluft in die Beatmungsluft gelangen kann.

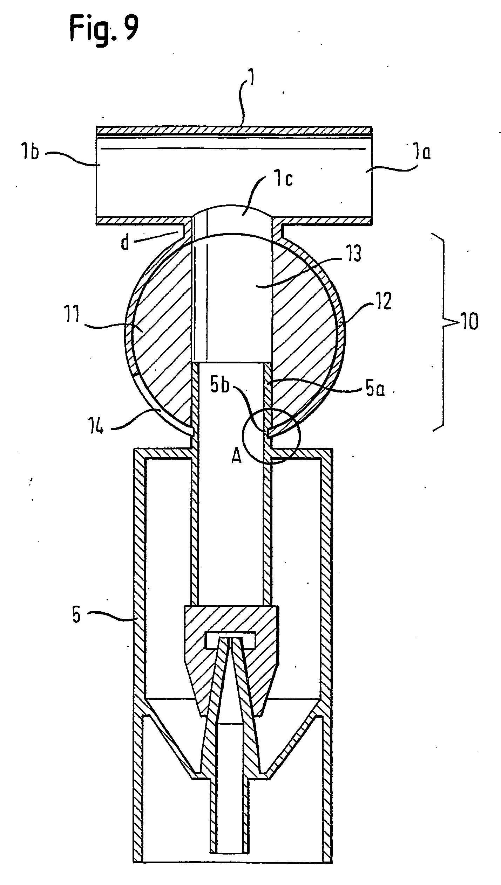

Das Verschlussorgan 11 umfasst ein Verschlussorganöffnung 13, in die ein Abgabestutzen 5a eines Verneblers 5 eingeführt werden kann. Figur 7 zeigt den Vernebler 5 bevor der Abgabestutzen 5a in die Verschlussorganöffnung 13 eingesteckt wurde; Figur 8 zeigt den Vernebler 5 nachdem der Abgabestutzen 5a in die Verschlussorganöffnung 13 eingesteckt wurde. Um das Einstecken zu ermöglichen, besitzt das Verschlussorgangehäuse 12 eine Verschlussorgangehäuseöffnung 14, die so geformt ist, dass der Abgabestutzen 5a des Verneblers 5 durch die Verschlussorgangehäuseöffnung 14 hindurch in die Verschlussorganöffnung 13 eingeführt und der Abgabestutzen 5a entlang eines Kreisabschnitts derart bewegt werden kann, dass das Verschlussorgan 11 in dem Verschlussorgangehäuse 12 gedreht wird. Die Drehbewegung wird vorgegeben durch eine zylindrische oder kugelförmige Gestaltung sowohl des Verschlussorgans 11 als auch des Verschlussorgangehäuses 12. In den Figuren 7 bis 9 sind Querschnitte gezeigt, die sowohl einer flachen zylindrischen Gestalt als auch einer kugelförmigen Gestalt von Verschlussorgan 11 und Verschlussorgangehäuse 12 entsprechen. Wie aus den Figuren 7 bis 9 ersichtlich, ist die Verschlussorgangehäuseöffnung 14 ein in der Mantel- bzw. Kugeloberfläche verlaufendes Langloch, so dass ein zylindrischer Abgabestutzen 5a eines Verneblers 5 eingeführt und in der Verschlussorgangehäuseöffnung 14 verschwenkt werden kann. Wie sich aus Figur 9 ergibt, ist in der verschwenkten Stellung die Verschlussorganöffnung 13 dem Anschlussstutzen 1c gegenüberliegend angeordnet, so dass der Abgabestutzen 5a des Verneblers 5 mit dem Anschlussstutzen 1c ausgerichtet ist. Das von dem Vernebler erzeugte Aerosol gelangt so auf ungestörtem Weg aus dem Vernebler 5 durch den Abgabestutzen 5a und den Anschlussstutzen 1c in die Atemluftführungseinrichtung 1 der erfindungsgemäßen Vernebleranschlussvorrichtung.

Beim Entfernen des Verneblers 5 wird zunächst der Vernebler 5 mit dem Abgabestutzen 5a in die in Figur 8 gezeigte Stellung bewegt, wodurch das Verschlussorgan 11 die dritte Anschlusseinrichtung 1c verschließt, wie in Figur 8 gezeigt ist. Dann wird der Abgabestutzen 5a des Verneblers 5 aus der Verschlussorganöffnung 13 herausgezogen, wie Figur 7 zeigt. Erfindungsgemäß ist dann die dritte Anschlusseinrichtung 1c verschlossen, so dass weder Beatmungsluft in die Umgebung noch Umgebungsluft in die Beatmungsluft gelangen kann.

Um zu vermeiden, dass bei der in Figur 9 gezeigten Stellung der Abgabestutzen 5a des Verneblers 5 unbeabsichtigt aus der Verschlussorganöffnung 13 herausgezogen wird, ist die Verschlussorgangehäuseöffnung 14 in einem dem Anschlussstutzen 1c gegenüberliegenden Bereich enger ausgeführt, als in einem Bereich, der mit der Position der Verschlussorganöffnung 13 in der in Figur 7 oder 8 gezeigten Stellung ausgerichtet ist. Der Außendurchmesser des Abgabestutzen 5a ist größer als die Breite der Verschlussorgangehäuseöffnung 14 in dem verengten Bereich. Damit der Abgabestutzen 5a des Verneblers 5 in den engeren Bereich der Verschlussorgangehäuseöffnung 14 geschwenkt werden kann, besitzt der Abgabestutzen 5a eine Einkerbung 5b, die die Außenabmessung des Abgabestutzens 5a ausreichend verringert, um in den engeren Bereich der Verschlussorgangehäuseöffnung 14 eingeschoben werden zu können. In Figur 9 ist an der Stelle A erkennbar, wie das Verschlussorgangehäuse 12 in die Einkerbung 5b des Abgabestutzens 5a eingreift, wodurch verhindert wird, dass der Abgabestutzen 5a des Verneblers 5 in der in Figur 9 gezeigten Stellung aus der Verschlussorganöffnung 13 herausgezogen wird. Da sich der enge Bereich der Verschlussorgangehäuseöffnung 14 so weit erstreckt, dass der Eingriff des Verschlussorgangehäuses 12 in die Einkerbung 5b des Abgabestutzens 5a erst aufgehoben ist, wenn das Verschlussorgan 11 die in Figur 8 gezeigte Position erreicht hat, ist sichergestellt, dass auch in einer Zwischenstellung zwischen der Verneblungsstellung des Verschlussorgans 11 gemäß Figur 9 und der Verschließstellung des Verschlussorgans 11 gemäß Figur 8 der Abgabestutzen 5a des Verneblers aus der Verschlussorganöffnung 13 nicht herausgezogen werden kann.

Wie in Figur 9 bereits angedeutet, ist der Abstand d zwischen der Verschlusseinrichtung 10 und der Atemluftführungseinrichtung 1 der erfindungsgemäßen Vernebleranschlussvorrichtung nahezu beliebig verkürzbar. Wenn der Abstand d minimiert wird, d.h. wenn die Verschlusseinrichtung 10 unmittelbar neben der Atemluftführungseinrichtung 1 angeordnet ist, wird erreicht, dass im geschlossenen Zustand praktisch kein Totvolumen entsteht und dass die Beatmungsluft nicht beeinträchtigt wird, die durch die Atemluftführungseinrichtung 1 strömt. Auch für das Aerosol, das über den Abgabestutzen 5a aus dem Vernebler in die Beatmungsluft strömt, ist der Strömungsweg minimiert, wenn der Abstand d so weit wie möglich verkürzt wird.

### 4. Ausführungsbeispiel

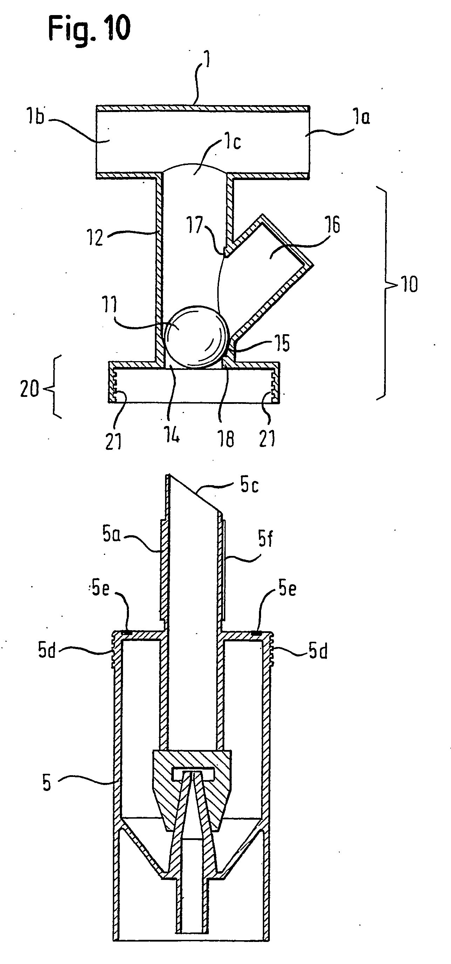

Die Figuren 10 bis 13 zeigen ein viertes Ausführungsbeispiel einer erfindungsgemäßen Vernebleranschlussvorrichtung für Beatmungsgeräte oder dergleichen.

Aus den Figuren 10 bis 13 ergibt sich zunächst, dass auch das vierte Ausführungsbeispiel, wie die anderen Ausführungsbeispiele, eine Atemluftführungseinrichtung 1, beispielsweise das in Figur 10 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

Wie den Figuren 10 bis 13 erkennbar, ist an einer dritten Anschlusseinrichtung 1c, beispielsweise dem in den Figuren 10 bis 13 erkennbaren Rohrstück, das in die Atemluftführungseinrichtung 1 einmündet, erfindungsgemäß eine Verschlusseinrichtung 10 angeordnet. Die Verschlusseinrichtung 10 des vierten Ausführungsbeispiels gemäß der Erfindung umfasst ein Verschlussorgan 11, das in einem Verschlussorgangehäuse 12 angeordnet ist. Figur 10 zeigt das Verschlussorgan 11 in der den Strömungsweg der dritten Anschlusseinrichtung 1c verschließenden Stellung. Wie sich aus Figur 10 ergibt befindet sich das Verschlussorgan 11 dabei in einer Position, die die eine Verschlussorgangehäuseöffnung 14 des Verschlussorgangehäuses 12 verschließt, so dass durch den Anschlussstutzen 1c weder Beatmungsluft in die Umgebung noch Umgebungsluft in die Beatmungsluft gelangen kann. Dazu ist der Verschlussbereich 15 des Verschlussorgangehäuses 12, in dem sich das Verschlussorgan 11 in der verschließenden Stellung befindet, im Sinne eines Ventilsitzes 15 ausgestaltet, was zu einem dichten Verschluss der Verschlussorgangehäuseöffnung 14 führt. Bei dem hier gezeigten vierten Ausführungsbeispiel der Erfindung ist das Verschlussorgan 11 als Kugel ausgebildet, die sich bei einem zylindrischen Verschlussorgangehäuse 12 beispielsweise auf einem ringförmigen Verschlussbereich 15 abstützt. Sowohl die Gravitation als auch der durch die Beatmungsluft bewirkte Überdruck im Inneren des Verschlussorgangehäuses 12 zu einem sicheren Sitz der Kugel 11 auf dem Ringbereich 15 und damit zu einem zuverlässigen Verschluss führt.

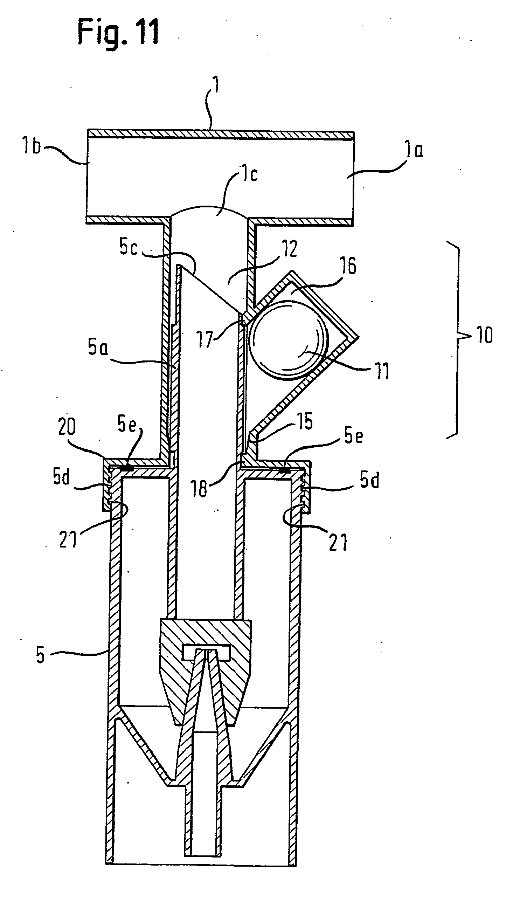

Wie in Figur 10 bereits angedeutet, wird der Abgabestutzen 5a eines Verneblers 5 in die Verschlussorgangehäuseöffnung 14 eingesteckt, um den Vernebler anzuschließen. Der Abgabestutzen 5a weist einen Einwirkbereich 5c auf, beispielsweise in Form eines abgeschrägten oder abgerundeten Stirnbereichs, der auf das Verschlussorgan 11 beim Einstecken des Abgabestutzens 5a in die Verschlussorgangehäuseöffnung 14 einwirkt und das Verschlussorgan 11 aus der Verschlussstellung bewegt, so dass das Verschlussorgan 11 in einen Verdrängungsbereich 16 bewegt wird, der in dem Verschlussorgangehäuse 12 zur Aufnahme des Verschlussorgans 11 vorgesehen ist. Dorthin wird das Verschlussorgan 11 durch den Abgabestutzen 5a des Verneblers 5 beim Einstecken verschoben. Durch die Gestalt des Einwirkbereichs 5c des Abgabestutzens 5a ist sichergestellt, dass das Verschlussorgan 11 den Verdrängungsbereich 16 gelangt. Zusätzlich kann ein Ablenkvorsprung 17 in dem Verschlussorgangehäuse 12 vorgesehen sein, der verhindert, dass das Verschlussorgan 11 in den Anschlussstutzen 1c gelangt. Figur 11 zeigt die Lage des Abgabestutzens 5a nach dem Einstecken in das Verschlussorgangehäuse 12 und des Verschlussorgans 11 in dem Verdrängungsbereich 16.

Ferner zeigen die Figuren 10 und 11 eine Verneblerhalteeinrichtung 20, die erste Rastelemente 21 umfasst, die mit zweiten Rastelementen 5d zusammenwirken, die an dem Vernebler 5 vorgesehen sind. Wenn der Vernebler 5 an der Anschlussvorrichtung gemäß dem vierten Ausführungsbeispiel angebracht wird, gelangen die Rastelemente 5d und 21 in Eingriff und führen zu einer Halterung des Verneblers. Die Wirkung der Rastelemente ist derart, dass der Vernebler durch eine Krafteinwirkung, die von einem Menschen zerstörungsfrei aufgebracht werden kann, wieder von der Anschlussvorrichtung gelöst werden kann. Im Übrigen ist die Rastwirkung ausreichend, um den Vernebler sicher zu fixieren.

Daneben zeigen die Figuren 10 und 11, dass an dem Vernebler 5 ein oder mehrere Dichtelemente 5e, beispielsweise ein Dichtring, vorgesehen sein können, die mit der Halteeinrichtung 20 zusammen wirken, um im befestigten Zustand im Bereich des Verneblers 5 eine Abdichtung des Verschlussorgangehäuses 12 zur Umgebung zu bewerkstelligen.

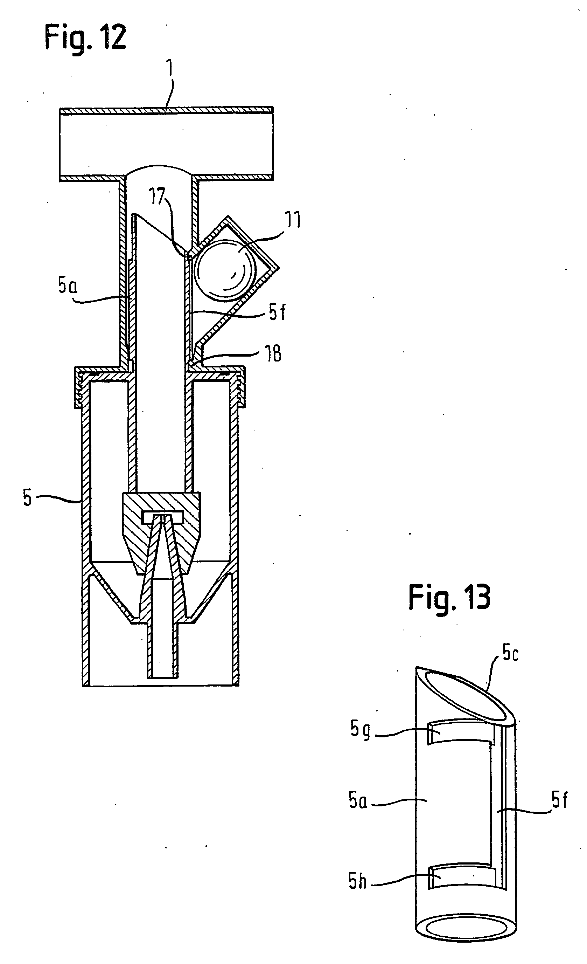

Damit der Abgabestutzen 5a nur so eingesteckt werden kann, dass der Einwirkbereich 5c das Verschlussorgan 11 in den Verdrängungsbereich 16 hinein bewegt, ist an dem Abgabestutzen 5a, wie die Figuren 12 und 13 zeigen, vorzugsweise eine Ausrichtnut 5f vorgesehen, die mit einem Ausrichtvorsprung 18 derart zusammenwirkt, dass der Abgabestutzen 5a nur in die Verschlussorgangehäuseöffnung 14 eingesteckt werden kann, wenn der Ausrichtvorsprung 18 in der Ausrichtnut 5f positioniert ist. Die Ausrichtnut 5f kann überdies den Ablenkvorsprung 17 aufnehmen, wenn der Abgabestutzen 5a sich im eingesteckten Zustand bis dahin erstreckt, wie Figur 12 zeigt. Die Ausrichtnut 5f ist auch in Figur 13 dargestellt, die den Aerosolabgabestutzen 5a allein zeigt.

In Figur 13 ist zusätzlich gezeigt, dass in dem Abgabestutzen 5a auch erste und zweite Halterungsnuten 5g und 5h vorgesehen sein können, mit deren Hilfe der Vernebler 5 alternativ oder ergänzend zu den Rastelementen, die in den Figuren 10, 11 und 12 gezeigt und oben beschrieben sind, im eingesteckten Zustand gehaltert werden kann. Denn die Halterungsnuten 5g und 5h wirken mit dem Ablenkvorsprung 17 bzw. dem Ausrichtvorsprung 18 in dem Verschlussorgangehäuse 12 zusammen, wenn nach dem Einstecken des Abgabestutzens 5a, das mit Hilfe der Ausrichtnut 5d und den beiden Vorsprüngen 17 und 18 ausgerichtet erfolgt, der Vernebler 5 und damit der Abgabestutzen 5a gedreht wird, so dass die beiden Vorsprünge 17 und 18 in jeweils eine der beiden Halterungsnuten 5g und 5h gelangen.

### 5. Ausführungsbeispiel

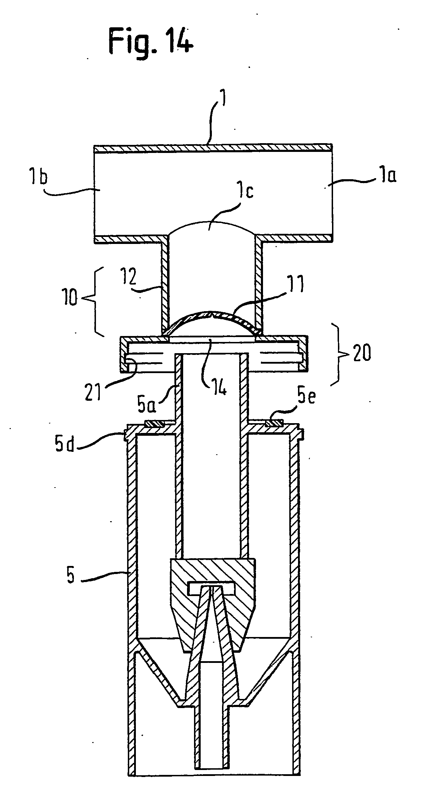

Die Figuren 14 bis 17 zeigen ein fünftes Ausführungsbeispiel einer erfindungsgemäßen Vernebleranschlussvorrichtung für Beatmungsgeräte oder dergleichen.

Aus den Figuren 14 bis 17 ergibt sich zunächst, dass auch das fünfte Ausführungsbeispiel, wie die anderen Ausführungsbeispiele, eine Atemluftführungseinrichtung 1, beispielsweise das in Figur 14 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

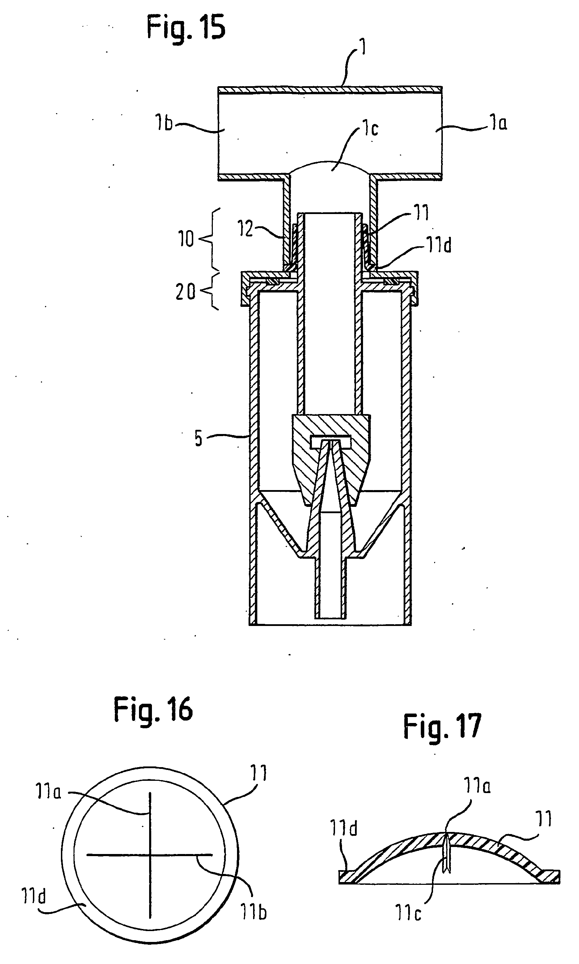

Wie den Figuren 14 bis 17 erkennbar, ist an einer dritten Anschlusseinrichtung 1c, beispielsweise dem in den Figuren 14 und 15 erkennbaren Rohrstück, das in die Atemluftführungseinrichtung 1 einmündet, erfindungsgemäß eine Verschlusseinrichtung 10 angeordnet. Die Verschlusseinrichtung 10 des fünften Ausführungsbeispiels gemäß der Erfindung umfasst ein Verschlussorgan 11, das in einem Verschlussorgangehäuse 12 angeordnet ist. Die Figur 14 zeigt das Verschlussorgan 11 in der den Strömungsweg der dritten Anschlusseinrichtung 1c verschließenden Stellung. Bei dem Verschlussorgan des fünften Ausführungsbeispiels handelt es sich um ein kalottenartig geformtes Ventilelement 11, das mittig zwei über Kreuz verlaufende Schlitze 11a und 11b aufweist, was in Figur 16 dargestellt ist. In der vergrößerten Darstellung der Figur 17 ist zu erkennen, dass auf der konkaven Seite der Ventilkalotte an den Schnitten eine V-förmige Kerbe 11c ausgebildet ist, durch die ein optimaler Verschluss durch das Ventilelement 11 gewährleistet wird, wenn auf der konvexen Seite der Ventilkalotte 11 ein Überdruck existiert. Dadurch wird erreicht, dass das kalottenförmige Ventilelement 11 eine Verschlussorgangehäuseöffnung 14 des Verschlussorgangehäuses 12 verschließt, so dass durch den Anschlussstutzen 1c weder Beatmungsluft in die Umgebung noch Umgebungsluft in die Beatmungsluft gelangen kann. Das Ventilelement 11 ist am Rand 11d geeignet an der Verschlusseinrichtung 10 fixiert.

Wie in Figur 14 bereits angedeutet, wird der Abgabestutzen 5a eines Verneblers 5 in die Verschlussorgangehäuseöffnung 14 eingesteckt, um den Vernebler anzuschließen. Der Abgabestutzen 5a kann aufgrund der Schlitze 11a und 11b das Ventilelement 11 durchdringen, wodurch der Strömungsweg für das von dem Vernebler erzeugte Aerosol freigegeben ist. Wird der Abgabestutzen 5a des Verneblers 5 wieder herausgezogen, schließt sich das Ventilelement 11 aufgrund seiner Elastizität und verschließt den Strömungsweg, wie Figur 14 zeigt.

Ferner zeigen die Figuren 14 und 15 eine Verneblerhalteeinrichtung 20, die erste Rastelemente 21 umfasst, die mit zweiten Rastelementen 5d zusammenwirken, die an dem Vernebler 5 vorgesehen sind. Wenn der Vernebler 5 an der Anschlussvorrichtung gemäß dem fünften Ausführungsbeispiel angebracht wird, gelangen die Rastelemente 5d und 21 in Eingriff und führen zu einer Halterung des Verneblers. Die Wirkung der Rastelemente ist derart, dass der Vernebler durch eine Krafteinwirkung, die von einem Menschen zerstörungsfrei aufgebracht werden kann, wieder von der Anschlussvorrichtung gelöst werden kann. Im Übrigen ist die Rastwirkung ausreichend, um den Vernebler sicher zu fixieren. Anstelle der Rastelemente ist auch ein Gewinde oder eine andere geeignete Fixierung einsetzbar.

Daneben zeigen die Figuren 14 und 15, dass an dem Vernebler 5 ein oder mehrere Dichtelemente 5e, beispielsweise ein Dichtring, vorgesehen sein können, die mit der Halteeinrichtung 20 zusammen wirken, um im befestigten Zustand im Bereich des Verneblers 5 eine Abdichtung des Verschlussorgangehäuses 12 zur Umgebung zu bewerkstelligen.

### 6. Ausführungsbeispiel

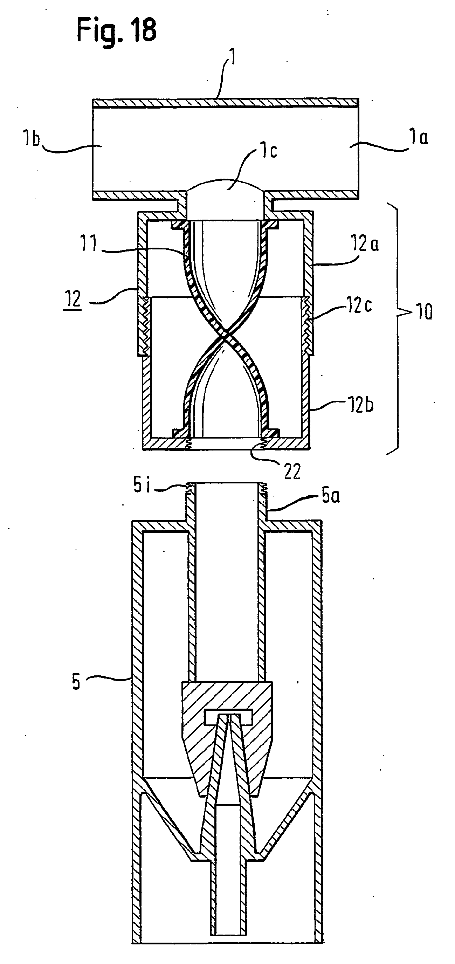

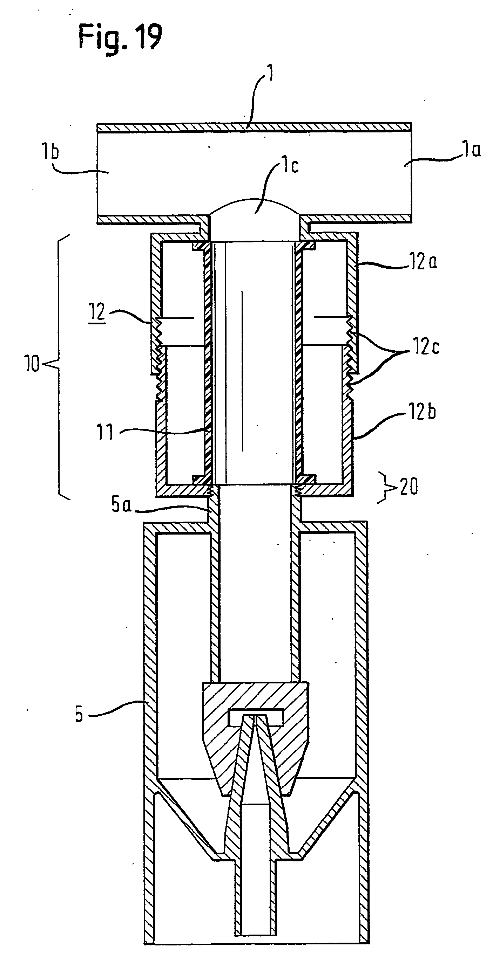

Die Figuren 18 und 19 zeigen ein sechstes Ausführungsbeispiel einer erfindungsgemäßen Vernebleranschlussvorrichtung für Beatmungsgeräte oder dergleichen.

Aus den Figuren 18 und 19 ergibt sich zunächst, dass auch das sechste Ausführungsbeispiel, wie die anderen Ausführungsbeispiele, eine Atemluftführungseinrichtung 1, beispielsweise das in Figur 18 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist..Eine zweite Anschlusseinrichtung 1b dient dem Anschluss einer Beatmungsluft abführenden Atemluftleitung, in der Regel ein Schlauch, der die Beatmungsluft dem Patienten zuführt.

Wie den Figuren 18 und 19 erkennbar, ist an einer dritten Anschlusseinrichtung 1c, beispielsweise dem in den Figuren 18 und 19 erkennbaren Rohrstück, das in die Atemluftführungseinrichtung 1 einmündet, erfindungsgemäß eine Verschlusseinrichtung 10 angeordnet. Die Verschlusseinrichtung 10 des sechsten Ausführungsbeispiels gemäß der Erfindung umfasst ein Verschlussorgan 11, das in einem Verschlussorgangehäuse 12 angeordnet ist. Die Figur 18 zeigt das Verschlussorgan 11 in der den Strömungsweg der dritten Anschlusseinrichtung 1c verschließenden Stellung. Bei dem Verschlussorgan des sechsten Ausführungsbeispiels handelt es sich um ein Schlauchstück 11, dass an einem Ende an einem ersten Gehäuseteil 12a des Verschlussorgangehäuses 12 und an dem anderen Ende mit einem zweiten Gehäuseteil 12b des Verschlussorgangehäuses 12 fest verbunden ist. In der verschließenden Stellung ist das Schlauchstück 11 soweit tordiert, dass das Schlauchstück 11 eine Verschlussorgangehäuseöffnung 14 des Verschlussorgangehäuses 12 verschließt, so dass durch den Anschlussstutzen 1c weder Beatmungsluft in die Umgebung noch Umgebungsluft in die Beatmungsluft gelangen kann.

Die beiden Gehäuseteile 12a und 12b des Verschlussorgangehäuses 12 sind gegeneinander verdrehbar miteinander verbunden, beispielsweise mit Hilfe von zwei Gewindebereichen 12c, die an einander zugewandten Bereichen der beiden Gehäuseteile 12a und 12b ausgebildet sind. Dadurch ist das zweite Gehäuseteil 12b, das nicht fest mit dem Anschlussstutzen 1c verbunden ist, gegenüber dem ersten Gehäuseteil 12a, das fest mit dem Anschlussstutzen 1c verbunden ist, verdrehbar, so dass das Schlauchstück 11 aus seiner tordierten und damit den Strömungsweg verschließenden Position in eine den Strömungsweg freigebende Position verdreht werden kann.

Dazu und zur Fixierung eines Verneblers 5 ist an der Verschlussorgangehäuseöffnung 14 ein Innengewinde 22 vorgesehen, in das ein Außengewinde 5i einschraubbar ist, das an einem Abgabestutzen 5a des Verneblers 5 ausgebildet ist. Wird der Abgabestutzen 5a an der Verschlussorgangehäuseöffnung 14 festgeschraubt, wird der Vernebler 5 zunächst sicher mit der erfindungsgemäßen Vernebleranschlussvorrichtung verbunden. Das Innengewinde 22 und das Außengewinde 5i bilden zusammen eine Verneblerhalteeinrichtung 20. Wird die Drehbewegung, die zur Halterung erforderlich ist, fortgesetzt, wird der zweite Gehäuseteil 12b gegen den ersten Gehäuseteil 12a verdreht, so dass das Schlauchstück von der in Figur 18 gezeigten tordierten Stellung in die in Figur 19 gezeigten geöffnete Stellung überführt wird. Wird der Vernebler 5 wieder von der Vernebleranschlussvorrichtung getrennt, wird zunächst eine Drehbewegung ausgeführt, die das Schlauchstück 11 tordiert, wie in Figur 18 gezeigt. Erst anschließend wird der Vernebler 5 gelöst, indem der Abgabestutzen 5a aus der Verschlussorgangehäuseöffnung 14 herausgeschraubt wird.

### 7. Ausführungsbeispiel

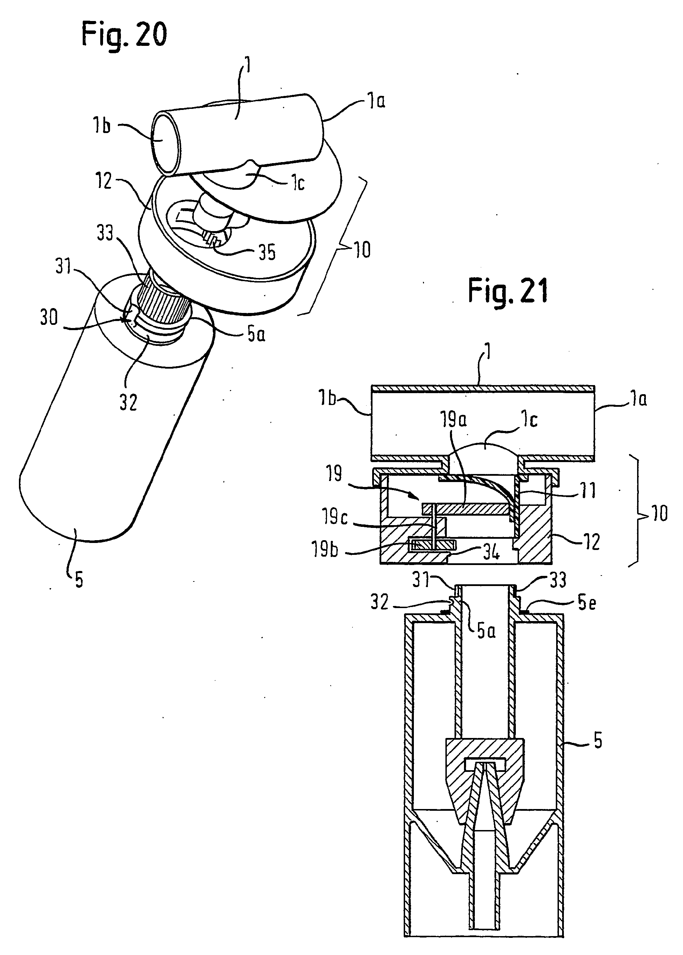

Die Figuren 20 bis 22 zeigen ein siebtes Ausführungsbeispiel einer erfindungsgemäßen Vernebleranschlussvorrichtung für Beatmungsgeräte oder dergleichen.

Aus den Figuren 21 bis 23 ergibt sich zunächst, dass auch das siebte Ausführungsbeispiel, wie die anderen Ausführungsbeispiele, eine Atemluftführungseinrichtung 1, beispielsweise das in Figur 20 gezeigte Rohrstück 1 aufweist, das in eine Leitung für die von einem Beatmungsgerät herangeführte und einem Patienten zugeführte Beatmungsluft eingesetzt wird. Eine erste Anschlusseinrichtung 1a, im einfachsten Fall eines der beiden Rohrenden, dient dem Anschluss einer Beatmungsluft heranführenden Atemluftleitung, in der Regel ein Schlauch, der mit einem Beatmungsgerät verbunden ist. Eine zweite Anschlusseinrichtung 1b dient dem Anschluss einer Beatmungsluft abführenden Atemluftleitung, in der Regel ein Schlauch, der die Beatmungsluft dem Patienten zuführt.

Wie den Figuren 20 bis 22 erkennbar, ist an einer dritten Anschlusseinrichtung 1c, beispielsweise dem in den Figuren 20 bis 22 erkennbaren kurzen Rohrstück, das in die Atemluftführungseinrichtung 1 einmündet, erfindungsgemäß eine Verschlusseinrichtung 10 angeordnet. Die Verschlusseinrichtung 10 des siebten Ausführungsbeispiels gemäß der Erfindung umfasst ein Verschlussorgan 11, das in einem Verschlussorgangehäuse 12 angeordnet ist. Die Figur 21 zeigt das Verschlussorgan 11 in der den Strömungsweg der dritten Anschlusseinrichtung 1c verschließenden Stellung. Bei dem Verschlussorgan des siebten Ausführungsbeispiels handelt es sich um ein kurzes Schlauchstück 11, dass an einem Ende mit dem Verschlussorgangehäuse 12 verbunden ist. In der verschließenden Stellung, die Figur 21 zeigt, klemmt ein Klemmmmechanismus 19 das Schlauchstück 11 soweit zusammen, dass durch den Anschlussstutzen 1c weder Beatmungsluft in die Umgebung noch Umgebungsluft in die Beatmungsluft gelangen kann.

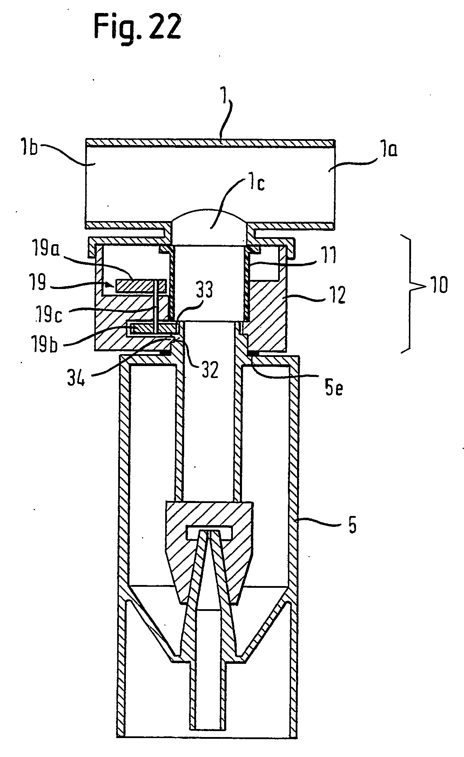

Der Klemmmechanismus 19 umfasst bei dem in den Figuren 21 und 22 gezeigten siebten Ausführungsbeispiel ein Klemmelement 19a, das unmittelbar auf den Schlauch 11 einwirkt und ihn in der verschließenden Stellung einklemmt, ein Betätigungselement 19b, mit dessen Hilfe das Klemmelement 19a betätigt wird, und ein Verbindungselement 19c, das eine Verbindung zwischen dem Klemmelement 19a und dem Betätigungselement 19b herstellt. Wenn auf das Betätigungselement 19b eine Kraft einwirkt, die das Betätigungselement dreht, wird die Kraft und damit die Drehbewegung aufgrund der Wirkung des Verbindungselements 19c auf die Klemmeinrichtung 19a übertragen. Auf diese Weise wird das Klemmelement 19a aus der verschließenden Position, die in Figur 21 gezeigt ist, in die geöffnete Position überführt, die Figur 22 zeigt. Wie in Figur 22 dargestellt, ist dann der Strömungsweg durch den Anschlussstutzen 1c frei für die Zuführung eines Aerosol in den Beatmungsluftstrom.

Zur Fixierung eines Verneblers 5 ist an dem Abgabestutzen 5a des Verneblers 5 ein Bajonett-Verbinder 30 mit einer in Längsrichtung des Abgabestutzens 5a verlaufenden Einstecknut 31 und einer am Umfang des Abgabestutzens 5a verlaufenden Verriegelungsnut 32 vorgesehen. An der Verschlussorgangehäuseöffnung 14 ist ein ein entsprechender Verriegelungszapfen 34 vorgesehen, der in die Einstecknut 31 eingeführt und durch Verdrehen des Verneblers 5 in die Verriegelungsnut 32 bewegt wird, um den Vernebler 5 sicher an der Verschlussorgangehäuseöffnung 14 zu haltern. Bei der Drehbewegung des Verneblers 5 wird über eine verzahnte Oberläche 33 am Abgabestutzen 5a die Drehbewegung auf das Betätigungselement 19b übertragen, das eine entsprechende Verzahnung 35 aufweist. Das Verbindungselement 19c überträgt die Drehbewegung auf das Betätigungselement 19a, das aus der verschließenden Stellung (Figur 21) in die geöffnete Stellung (Figur 22) geschwenkt wird. Das Schlauchstück 11 öffnet sich aufgrund der nun nicht mehr gegebenen Klemmeinwirkung des Klemmelements 19a wegen seiner Eigenelastizität und gibt den Strömungsweg für das Aerosol frei.

Wenn der Vernebler 5 wieder von der Vernebleranschlussvorrichtung getrennt, wird zunächst eine Drehbewegung ausgeführt, die das Betätigungselement 19b zurückdreht, wodurch über das Verbindungselement 19c auch das Betätigungselement 19a in die Klemmstellung zurückbewegt wird und den Schlauch 11 verschließend einklemmt. Erst anschließend wird der Vernebler 5 von der erfindungsgemäßen Vernebleranschlussvorrichtung gelöst, indem der Abgabestutzen 5a von dem Bajonett-Verbinder 30 der Verschlussorgangehäuseöffnung 14 getrennt wird.

Grundsätzlich eignet sich die erfindungsgemäße Vernebleranschlussvorrichtung für jeden Verneblertyp, also nicht nur einen in den Figuren angedeuteten Düsenvernebler, bei dem ein Aerosol mit Hilfe einer Druckluftdüse erzeugt wird, die eine Flüssigkeit aus einem Flüssigkeitsvorratsbehälter vernebelt. Verwendbar sind auch Ultraschallvernebler aber insbesondere Membranvernebler, bei denen eine Flüssigkeit durch eine poröse Membran hindurch vernebelt wird, wenn die Membran durch ein Piezoelement in Schwingungen versetzt wird. Bei den zuletzt erwähnten Membranverneblern kann vorgesehen werden, dass die dritte Anschlusseinrichtung der Atemluftführungseinrichtung im Gebrauch nach oben gerichtet ist und sich dementsprechend das Verschlussorgangehäuse sowie alle anderen Komponenten oberhalb der Atemluftführungseinrichtung befindet. Die Anordnung oberhalb der Atemluftführungseinrichtung und damit oberhalb der Beatmungsluftleitung kann auch bei anderen Verneblerarten sinnvoll sein. In allen hier geschilderten Fällen ist ferner eine Gestaltung zweckdienlich, bei der die dritte Anschlusseinrichtung nicht senkrecht sondern schräg in die Atemluftführungseinrichtung einmündet, um Deposition weiter zu verringern. Denn eine schräge Gestaltung der dritten Anschlusseinrichtung führt zu einer Einleitung des Aerosol in die Atemluftführungseinrichtung in Richtung des Atemstroms.

## Patentansprüche

1. Vernebleranschlussvorrichtung für Beatmungsgeräte oder dergleichen mit
- einer Atemluftführungseinrichtung (1) mit
o einer ersten Anschlusseinrichtung (1a) für den Anschluss einer Atemluft heranführenden Atemluftleitung,
o einer zweiten Anschlusseinrichtung (1b) für den Anschluss einer Atemluft abführenden Atemluftleitung; und
o einer dritten Anschlusseinrichtung (1c) für den Anschluss eines Verneblers (5), und mit
einer Verschlusseinrichtung (10), die an der dritten Anschlusseinrichtung (1c) angeordnet ist und die ein Verschlussorgan (11) und ein Verschlussorgangehäuse (12) aufweist, wobei die Freigabe bzw. das Verschließen des Strömungswegs für das Aerosol durch die dritte Anschlusseinrichtung (1c) über eine Einwirkung des Verneblers (5) oder eines Teiles (5a) davon auf die Verschlusseinrichtung (10) erfolgt, **dadurch gekennzeichnet, dass** das Verschlussorgan (11) sowohl in einer den Strömungsweg für ein von einem angeschlossenen Vernebler erzeugtes Aerosol durch die dritte Anschlusseinrichtung (1c) freigebenden Stellung als auch in einer den Strömungsweg für ein von einem angeschlossenen Vernebler erzeugtes Aerosol durch die dritte Anschlusseinrichtung (1c) verschließenden Stellung im Verschlussorgangehäuse (12) angeordnet ist, wobei sich das Verschlussorgan (11) in der freigebenden Stellung vollständig außerhalb des Strömungsweges durch die dritte Anschlusseinrichtung (1c) befindet.

2. Vernebleranschlussvorrichtung nach Anspruch 1, **dadurch gekennzeichnet, dass** die Atemluftführungseinrichtung (1) ein erstes Rohrstück ist, dass die erste und zweite Anschlusseinrichtung (1a, 1b) die Enden des ersten Rohrstücks (1) sind und die dritte Anschlusseinrichtung (1c) ein zweites Rohrstück (1c) ist, das in das erste Rohrstück (1) mündet.

3. Vernebleranschlussvorrichtung nach Anspruch 2, **dadurch gekennzeichnet, dass** das zweite Rohrstück (1c) im wesentlichen senkrecht zu dem ersten Rohrstück (1) verläuft.

4. Vernebleranschlussvorrichtung nach einem der vorangegangenen Ansprüche, **dadurch gekennzeichnet, dass** die Verschlusseinrichtung (10) ein Verschlussorgan (11) in einem Verschlussorgangehäuse (12) aufweist, in dem das Verschlussorgan (11) mit Hilfe des Verneblers (5) oder eines Teiles (5a) davon aus einer Stellung, in der das Verschlussorgan (11) den Strömungsweg eines von dem Vernebler erzeugten Aerosols in die Atemluftführungseinrichtung verschließt, in eine Stellung, in der das Verschlussorgan (11) den Strömungsweg eines von dem Vernebler erzeugten Aerosols in die Atemluftführungseinrichtung freigibt, und zurück bewegbar ist.

5. Vernebleranschlussvorrichtung nach Anspruch 4, **dadurch gekennzeichnet, dass** das Verschlussorgan (11) eine Verschlussorganöffnung (13) und das Verschlussorgangehäuse (12) eine Verschlussorgangehäuseöffnung (14) aufweist, durch die ein Aeroslabgabestutzen (5a) des Verneblers (5) in die Verschlussorganöffnung (13) einsteckbar und das Verschlussvorgang (11) durch Bewegen des Abgabestutzens (5a) in der Verschlussorgangehäuseöffnung (14) bewegbar ist.

6. Vernebleranschlussvorrichtung nach Anspruch 5, **dadurch gekennzeichnet, dass** die Verschlussorgangehäuseöffnung (14) einen ersten Bereich, dessen Abmessungen so ausgelegt ist, dass der Abgabestutzen (5a) des Verneblers (5) einsteckbar ist, und einen zweiten Bereich aufweist, dessen Abmessungen so ausgelegt ist, dass der Abgabestutzen (5a) nicht einsteckbar ist, und in den der eingesteckte Abgabestutzen (5a) nur bewegbar ist, wenn an dem Abgabestutzen (5a) eine Kerbe (5b) vorgesehen ist, die den Abgabestutzen (5a) an die lichte Weite des zweiten Bereichs anpasst.

7. Vernebleranschlussvorrichtung nach Anspruch 5 oder 6, **dadurch gekennzeichnet, dass** die Verschlussorganöffnung (13) eine Bohrung für die Aufnahme eine zylindrischen Abgabestutzens (5a) ist.

8. Vernebleranschlussvorrichtung nach Anspruch 5, 6 oder 7, **dadurch gekennzeichnet, dass** das Verschlussorgan (11) und das Verschlussorgangehäuse (12) im wesentlichen quaderförmig sind.

9. Vernebleranschlussvorrichtung nach Anspruch 5, 6 oder 7, **dadurch gekennzeichnet, dass** das Verschlussorgan (11) und das Verschlussorgangehäuse (12) im wesentlichen zylindrisch sind.

10. Vernebleranschlussvorrichtung nach Anspruch 9, **dadurch gekennzeichnet, dass** die Verschlussorganöffnung (13) sich parallel zur Zylinderachse erstreckt und die Verschlussorgangehäuseöffnung (14) in einer Stirnseite des Zylinders angeordnet ist.

11. Vernebleranschlussvorrichtung nach Anspruch 9, **dadurch gekennzeichnet, dass** die Verschlussorganöffnung (13) sich senkrecht zur Zylinderachse erstreckt und die Verschlussorgangehäuseöffnung (14) in der Mantelfläche des Zylinders angeordnet ist.

12. Vernebleranschlussvorrichtung nach Anspruch 5, 6 oder 7, **dadurch gekennzeichnet, dass** das Verschlussorgan (11) und das Verschlussorgangehäuse (12) im wesentlichen kugelförmig sind.

13. Vernebleranschlussvorrichtung nach Anspruch 4, **dadurch gekennzeichnet, dass** das Verschlussorgan (11) eine Kugel (10) ist und das Verschlussorgangehäuse (12) eine Verschlussorgangehäuseöffnung (14) aufweist, wobei die Kugel in dem Verschlussorgangehäuse (12) derart angeordnet ist, dass beim Einstecken eines Aerosolabgabestutzens (5a) des Verneblers (5) in die Verschlussorgangehäuseöffnung (14) die Kugel in einen Verdrängungsbereich (16) bewegt wird, der in dem Verschlussorgangehäuse (12) vorgesehen ist.

14. Vernebleranschlussvorrichtung nach Anspruch 13, **dadurch gekennzeichnet, dass** in dem Verschlussorgangehäuse (12) ein Ablenkvorsprung (17) vorgesehen ist, der verhindert, dass die Kugel (11) in die dritte Anschlusseinrichtung (1c) gelangt.

15. Vernebleranschlussvorrichtung nach Anspruch 13 oder 14, **dadurch gekennzeichnet, dass** in dem Verschlussorgangehäuse (12) ein Ausrichtvorsprung (18) vorgesehen ist, der das Einstecken eines Aerosolabgabestutzens (5a) eines Verneblers (5) nur in einer ausgerichteten Stellung zulässt, in der der Ausrichtvorsprung (18) in einer Ausrichtnut (5f) des Aerosolabgabestutzens (5a) des Verneblers (5) angeordnet ist.

16. Vernebleranschlussvorrichtung nach Anspruch 4, **dadurch gekennzeichnet, dass** das Verschlussorgan (11) ein kalottenartig geformtes Ventilelement (11) ist, das zumindest zwei einander kreuzende Schlitze (11a, 11b) aufweist und dessen konkave Oberfläche der Verschlussorgangehäuseöffnung (14) zugewandt ist.

17. Vernebleranschlussvorrichtung nach Anspruch 16, **dadurch gekennzeichnet, dass** auf der konkaven Seite des Ventilelements (11) an den Schlitzen (11a, 11b) eine V-förmige Kerbe ausgebildet ist.

18. Vernebleranschlussvorrichtung nach Anspruch 16 oder 17, **dadurch gekennzeichnet, dass** das Ventilelement (11) einen Rand (11c) für die Fixierung in/an dem Verschlussorgangehäuse (12) aufweist.

19. Vernebleranschlussvorrichtung nach Anspruch 4, **dadurch gekennzeichnet, dass** das Verschlussorgan (11) ein Schlauchstück (11) ist, das an dem Verschlussorgangehäuse (12) befestigt ist.

20. Vernebleranschlussvorrichtung nach Anspruch 19, **dadurch gekennzeichnet, dass** das Verschlussorgangehäuse (12) zwei Teile (12a, 12b) aufweist, das Schlauchstück (11) mit einem Ende an dem ersten Teil (12a), der mit der Atemluftführungseinrichtung (1) fest verbunden ist, und mit dem anderen Ende an dem zweiten Teil (12b) befestigt ist, der gegenüber dem ersten Teil (12a) verdrehbar ist, so dass das Schlauchstück aus einer durch Torsion geschlossenen Stellung in eine geöffnete Stellung und zurück bringbar ist.

21. Vernebleranschlussvorrichtung nach Anspruch 20, **dadurch gekennzeichnet, dass** die beiden Teile (12a, 12b) des Verschlussorgangehäuses (12) über einen Gewindebereich (12c) miteinander verbunden sind.

22. Vernebleranschlussvorrichtung nach Anspruch 19, **dadurch gekennzeichnet, dass** das Schlauchstück (11) durch einen Klemmmechanismus (19) verschließbar ist.

23. Vernebleranschlussvorrichtung nach Anspruch 22, **dadurch gekennzeichnet, dass** der Klemmmechanismus (19) ein Klemmelement (19a) umfasst, das auf das Schlauchstück einwirkt.

24. Vernebleranschlussvorrichtung nach Anspruch 23, **dadurch gekennzeichnet, dass** das Klemmelement (19a) mit einem Betätigungselement (19b) verbunden ist, so dass eine Drehbewegung des Betätigungselements auf das Klemmelement übertragen wird.

25. Vernebleranschlussvorrichtung nach Anspruch 24, **dadurch gekennzeichnet, dass** das Betätigungselement (19b) derart ausgelegt ist, dass der Aerosolabgabestutzen (5a) des Verneblers (5) auf das Betätigungselement einwirkt, so dass eine Drehbewegung des Aerosolabgabestutzens (5a) auf das Betätigungselement (19b) und weiter auf das Klemmelement (19b) übertragen wird.

26. Vernebleranschlussvorrichtung nach einem der vorangegangenen Ansprüche, **dadurch gekennzeichnet, dass** eine Halterungseinrichtung (20) vorgesehen ist, durch die der Vernebler (5) nach dem Anschließen gehaltert wird.

27. Vernebleranschlussvorrichtung nach Anspruch 26, **dadurch gekennzeichnet, dass** die Halteeinrichtung Rastelemente (21) oder ein Gewinde (22) für die Wechselwirkung mit Rastelementen (5d) bzw. ein Gewinde (5i) umfasst, die an dem Vernebler (5) vorgesehen sind.

28. Vernebleranschlussvorrichtung nach Anspruch 26, **dadurch gekennzeichnet, dass** die Halteeinrichtung Vorsprünge (17, 18) für die Wechselwirkung mit Haltenuten (5g, 5h) umfasst, die an dem Vernebler (5) vorgesehen sind.

29. Vernebleranschlussvorrichtung nach Anspruch 26, **dadurch gekennzeichnet, dass** die Halteeinrichtung einen Bajonett-Verbinder (30) mit einer in Längsrichtung des Abgabestutzens (5a) verlaufenden Einstecknut (31) und einer am Umfang des Abgabestutzens (5a) verlaufenden Verriegelungsnut (32) vorgesehen sind.

## Claims

1. Nebuliser connection device for respirators or the like having
a breathing air-guiding device (1) having
- a first connection device (1a) for connection of a breathing air-introducing breathing air pipe,
- a second connection device (1b) for connection of a breathing air-removing breathing air pipe; and
- a third connection device (1c) for connection of a nebuliser (5), and having
a closure device (10), which is arranged on the third connection device (1c) and which has a closure element (11) and a closure element housing (12), wherein the release or the closure of the flow path for the aerosol through the third connection device (1c) is effected via influence of the nebuliser (5) or a part (5a) thereof on the closure device (10), **characterised in that** the closure element (11) is arranged in the closure element housing (12) both in a position releasing the flow path for an aerosol produced by a connected nebuliser through the third connection device (1c) and in a position closing the flow path for an aerosol produced by a connected nebuliser through the third connection device (1c), wherein the closure element (11) is situated in the releasing position completely outside of the flow path through the third connection device (1c).

2. Nebuliser connection device according to claim 1, **characterised in that** the breathing air-guiding device (1) is a first tube piece, **in that** the first and second connection device (1a, 1b) are the ends of the first tube piece (1) and the third connection device (1c) is a second tube piece (1c) which leads into the first tube piece (1).

3. Nebuliser connection device according to claim 2, **characterised in that** the second tube piece (1c) runs essentially vertically to the first tube piece (1).

4. Nebuliser connection device according to one of the preceding claims, **characterised in that** the closure device (10) has a closure element (11) in a closure element housing (12), in which the closure element (11) can be moved with the aid of the nebuliser (5) or a part (5a) thereof from a position in which the closure element (11) closes the flow path of an aerosol produced by the nebuliser into the breathing air-guiding device, to a position in which the closure element (11) releases the flow path of an aerosol produced by the nebuliser into the breathing air-guiding device, and back.

5. Nebuliser connection device according to claim 4, **characterised in that** the closure element (11) has a closure element opening (13) and the closure element housing (12) has a closure element housing opening (14), through which an aerosol release connection (5a) of the nebuliser (5) can be inserted into the closure element opening (13), and the closure element (11) can be moved in the closure element housing opening (14) by moving the release connection (5a).

6. Nebuliser connection device according to claim 5, **characterised in that** the closure element housing opening (14) has a first region, the dimensions of which are designed so that the release connection (5a) of the nebuliser (5) can be inserted, and a second region, the dimensions of which are designed so that the release connection (5a) cannot be inserted, and in which the inserted release connection (5a) can only be moved when, at the release connection (5a), a notch (5b) is provided which adapts the release connection (5a) to the clear width of the second region.

7. Nebuliser connection device according to claim 5 or 6, **characterised in that** the closure element opening (13) is a bore for receiving a cylindrical release connection (5a).

8. Nebuliser connection device according to claim 5, 6 or 7, **characterised in that** the closure element (11) and the closure element housing (12) are essentially cuboid.

9. Nebuliser connection device according to claim 5, 6 or 7, **characterised in that** the closure element (11) and the closure element housing (12) are essentially cylindrical.

10. Nebuliser connection device according to claim 9, **characterised in that** the closure element opening (13) extends parallel to the cylinder axis and the closure element housing opening (14) is arranged in an end-face side of the cylinder.

11. Nebuliser connection device according to claim 9, **characterised in that** the closure element opening (13) extends vertically to the cylinder axis and the closure element housing opening (14) is arranged in the shell surface of the cylinder.

12. Nebuliser connection device according to claim 5, 6 or 7, **characterised in that** the closure element (11) and the closure element housing (12) are essentially spherical.

13. Nebuliser connection device according to claim 4, **characterised in that** the closure element (11) is a sphere (10) and the closure element housing (12) has a closure element housing opening (14), wherein the sphere is arranged in the closure element housing (12) such that when inserting an aerosol release connection (5a) of the nebuliser (5) into the closure element housing opening (14), the sphere is moved in a displacement region (16) which is provided in the closure element housing (12).

14. Nebuliser connection device according to claim 13, **characterised in that** a deflecting projection (17), which prevents the sphere (11) passing into the third connection device (1c), is provided in the closure element housing (12).

15. Nebuliser connection device according to claim 13 or 14, **characterised in that** an alignment projection (18), which permits the insertion of an aerosol release connection (5a) of a nebuliser (5) only in an aligned position, in which the alignment projection (18) is arranged in an alignment groove (5f) of the aerosol release connection (5a) of the nebuliser (5), is provided in the closure element housing (12).

16. Nebuliser connection device according to claim 4, **characterised in that** the closure element (11) is a valve element (11) shaped like a dome, which has at least two slots (11a, 11b) intersecting one another and the concave surface of which is facing the closure element housing opening (14).

17. Nebuliser connection device according to claim 16, **characterised in that** a V-shaped notch is formed on the concave side of the valve element (11) at the slots (11a, 11b).

18. Nebuliser connection device according to claim 16 or 17, **characterised in that** the valve element (11) has an edge (11c) for fixing in/to the closure element housing (12).

19. Nebuliser connection device according to claim 4, **characterised in that** the closure element (11) is a hose piece (11), which is attached to the closure element housing (12).

20. Nebuliser connection device according to claim 19, **characterised in that** the closure element housing (12) has two parts (12a, 12b), the hose piece (11) is attached by one end to the first part (12a), which is firmly connected to the breathing air-guiding device (1), and by the other end to the second part (12b), which can be rotated with respect to the first part (12a), so that the hose piece can be brought from a position which is closed by torsion to an open position and back.

21. Nebuliser connection device according to claim 20, **characterised in that** the two parts (12a, 12b) of the closure element housing (12) are connected to one another via a threaded region (12c).

22. Nebuliser connection device according to claim 19, **characterised in that** the hose piece (11) can be closed by a clamping mechanism (19).

23. Nebuliser connection device according to claim 22, **characterised in that** the clamping mechanism (19) comprises a clamping element (19a) which acts on the hose piece.

24. Nebuliser connection device according to claim 23, **characterised in that** the clamping element (19a) is connected to an actuating element (19b), so that a rotary movement of the actuating element is transferred to the clamping element.

25. Nebuliser connection device according to claim 24, **characterised in that** the actuating element (19b) is designed such that the aerosol release connection (5a) of the nebuliser (5) acts on the actuating element, so that a rotary movement of the aerosol release connection (5a) is transferred to the actuating element (19b) and also to the clamping element (19b).

26. Nebuliser connection device according to one of the preceding claims, **characterised in that** a mounting device (20) is provided, by means of which the nebuliser (5) is mounted after connection.

27. Nebuliser connection device according to claim 26, **characterised in that** the mounting device comprises locking elements (21) or a thread (22) for interaction with locking elements (5d) or a thread (5i) which are provided on the nebuliser (5).

28. Nebuliser connection device according to claim 26, **characterised in that** the mounting device comprises projections (17, 18) for interaction with mounting grooves (5g, 5h) which are provided on the nebuliser (5).

29. Nebuliser connection device according to claim 26, **characterised in that** the mounting device, a bayonet connector (30) with an insertion groove (31) running in longitudinal direction of the release connection (5a) and a locking groove (32) running on the periphery of the release connection (5a) are provided.

## Revendications

1. Ensemble raccord de nébuliseur pour appareils respiratoires ou analogues, comprenant :
- un dispositif de guidage d'air respiratoire (1), avec
o un premier ensemble raccord (1a), pour le raccordement d'une conduite d'air respiratoire amenant de l'air respiratoire,
o un deuxième ensemble raccord (1b), pour le raccordement d'une conduite d'air respiratoire évacuant de l'air respiratoire ; et
o un troisième ensemble raccord (1c), pour le raccordement d'un nébuliseur (5), et avec
un dispositif obturateur (10), disposé sur le troisième ensemble raccord (1c) et présentant un organe obturateur (11) et un boîtier d'organe obturateur (12), la libération ou l'obturation de la voie d'écoulement pour aérosol à travers le troisième ensemble raccord (1c) se faisant par la mise en action du nébuliseur (5) ou d'une partie (5a) de celui-ci sur le dispositif obturateur (10), la libération ou la fermeture de la voie d'écoulement pour l'aérosol à travers le troisième ensemble raccord (1c) se faisant par une mise en action du nébuliseur (5) ou d'une partie (5a) de celui-ci sur le dispositif obturateur (10), **caractérisé en ce que** l'organe obturateur (11), tant en une position libérant la voie d'écoulement à travers le troisième ensemble raccord (1c) pour un aérosol produit par un nébuliseur raccordé, qu'également en une position obturant la voie d'écoulement à travers le troisième ensemble raccord (1c) pour un aérosol produit par un nébuliseur raccordé, est disposé dans le boîtier d'organe obturateur (12), l'organe obturateur (11), à la position de libération se trouvant complètement hors de la voie d'écoulement passant par le troisième ensemble raccord (1c).

2. Ensemble raccord de nébuliseur selon la revendication 1, **caractérisé en ce que** le dispositif de guidage d'air respiratoire (1) est une première pièce tubulaire, **en ce que** le premier et le deuxième ensembles raccord (1a, 1b) sont les extrémités de la première pièce tubulaire (1), et le troisième ensemble raccord (1c) est une deuxième pièce tubulaire (1c) débouchant dans la première pièce tubulaire (1).

3. Ensemble raccord de nébuliseur selon la revendication 2, **caractérisé en ce que** la deuxième pièce tubulaire (1c) s'étend sensiblement perpendiculairement à la première pièce tubulaire (1).

4. Ensemble raccord de nébuliseur selon l'une des revendications précédentes, **caractérisé en ce que** le dispositif obturateur (10) présente un organe obturateur (11) dans un boîtier d'organe obturateur (12), dans lequel l'organe obturateur (11), à l'aide du nébuliseur (5) ou d'une partie (5a) de celui-ci, est déplaçable, d'une position à laquelle l'organe obturateur (11) obture la voie d'écoulement, dans le dispositif de guidage d'air respiratoire, d'un aérosol produit par le nébuliseur, en une position à laquelle l'organe obturateur (11) libère la voie d'écoulement, dans le dispositif de guidage d'air respiratoire, d'un aérosol produit par le nébuliseur, et retour.

5. Ensemble raccord de nébuliseur selon la revendication 4, **caractérisé en ce que** l'organe obturateur (11) présente une ouverture d'organe obturateur (13) et le boîtier d'organe obturateur (12) présente une ouverture de boîtier d'organe obturateur (14), à travers laquelle une tubulure de distribution d'aérosol (5a) du nébuliseur (5) est susceptible d'être enfichée dans l'ouverture d'organe obturateur (13), et l'organe obturateur (11) est déplaçable dans l'ouverture de boîtier d'organe obturateur (14), en procédant à un déplacement de la tubulure de distribution (5a).

6. Ensemble raccord de nébuliseur selon la revendication 5, **caractérisé en ce que** l'ouverture de boîtier d'organe obturateur (14) présente une première zone, dont les dimensions sont telles que la tubulure de distribution (5a) du nébuliseur (5) est susceptible d'être enfichée, et une deuxième zone dont les dimensions sont telles que la tubulure de distribution (5a) n'est pas susceptible d'être enfichée, et dans laquelle la tubulure de distribution (5a) ayant été enfichée n'est déplaçable que, lorsque sur la tubulure de distribution (5a), est prévue une entaille (5b) adaptant la tubulure de distribution (5a) à la largeur libre de la deuxième zone.

7. Ensemble raccord de nébuliseur selon la revendication 5 ou 6, **caractérisé en ce que** l'ouverture d'organe obturateur (13) est un perçage pour recevoir une tubulure de distribution (5a) cylindrique.

8. Ensemble raccord de nébuliseur selon la revendication 5, 6 ou 7, **caractérisé en ce que** l'organe obturateur (11) et le boîtier d'organe obturateur (12) sont à forme sensiblement parallélépipédique.

9. Ensemble raccord de nébuliseur selon la revendication 5, 6 ou 7, **caractérisé en ce que** l'organe obturateur (11) et le boîtier d'organe obturateur (12) sont sensiblement cylindriques.

10. Ensemble raccord de nébuliseur selon la revendication 9, **caractérisé en ce que** l'ouverture d'organe obturateur (13) s'étend parallèlement à l'axe de cylindre et l'ouverture de boîtier d'organe obturateur (14) est disposée dans une face frontale du cylindre.

11. Ensemble raccord de nébuliseur selon la revendication 9, **caractérisé en ce que** l'ouverture d'organe obturateur (13) s'étend perpendiculairement à l'axe de cylindre et l'ouverture de boîtier d'organe obturateur (14) est disposée dans la surface d'enveloppe du cylindre.

12. Ensemble raccord de nébuliseur selon la revendication 5, 6 ou 7, **caractérisé en ce que** l'organe obturateur (11) et le boîtier d'organe obturateur (12) sont sensiblement de forme sphérique.

13. Ensemble raccord de nébuliseur selon la revendication 4, **caractérisé en ce que** l'organe obturateur (11) est une sphère (10) et le boîtier d'organe obturateur (12) présente une ouverture de boîtier d'organe obturateur (14), la sphère étant disposée dans le boîtier d'organe obturateur (12), de manière que, lors de l'enfichage d'une tubulure de distribution d'aérosol (5a) du nébuliseur (5) dans l'ouverture de boîtier d'organe obturateur (14), la sphère soit déplacée dans une zone de refoulement (16) prévue dans le boîtier d'organe obturateur (12).

14. Ensemble raccord de nébuliseur selon la revendication 13, **caractérisé en ce que** dans le boîtier d'organe obturateur (12) est prévue une saillie de déviation (17), empêchant que la sphère (11) arrive dans le troisième ensemble raccord (1c).

15. Ensemble raccord de nébuliseur selon la revendication 13 ou 14, **caractérisé en ce que** dans le boîtier d'organe obturateur (12) est prévue une saillie orientatrice (18), permettant l'enfichage d'une tubulure de distribution d'aérosol (5a) d'un nébuliseur (5) uniquement dans une position orientée, à laquelle la saillie orientatrice (18) est disposée dans une rainure d'orientation (5f) de la tubulure de distribution d'aérosol (5a) du nébuliseur (5).

16. Ensemble raccord de nébuliseur selon la revendication 4, **caractérisé en ce que** l'organe obturateur (11) est un élément de soupape (11) en forme de calotte, présentant au moins deux fentes (11a, 11b), se croisant l'une l'autre et dont la surface concave est tournée vers l'ouverture de boîtier d'organe obturateur (14).

17. Ensemble raccord de nébuliseur selon la revendication 16, **caractérisé en ce que**, sur le côté concave de l'élément de soupape (11), sur les fentes (11a, 11b), est réalisée une entaille en forme de V.

18. Ensemble raccord de nébuliseur selon la revendication 16 ou 17, **caractérisé en ce que** l'élément de soupape (11) présente un bord (11c) pour la fixation dans/sur le boîtier d'organe obturateur (12).

19. Ensemble raccord de nébuliseur selon la revendication 4, **caractérisé en ce que** l'organe obturateur (11) est un morceau de tuyau (11) fixé sur le boîtier d'organe obturateur (12).

20. Ensemble raccord de nébuliseur selon la revendication 19, **caractérisé en ce que** le boîtier d'organe obturateur (12) présente deux parties (12a, 12b), le morceau de tuyau (11) est fixé, par une extrémité, sur la première partie (12a), qui est reliée rigidement au dispositif de guidage d'air respiratoire (1), et est fixé, par l'autre extrémité, sur la deuxième partie (12b) susceptible d'être tournée par rapport à la première partie (12a), de manière que le morceau de tuyau puisse être passé d'une position fermée par torsion en une position ouverte et retour.

21. Ensemble raccord de nébuliseur selon la revendication 20, **caractérisé en ce que** les deux parties (12a, 12b) du boîtier d'organe obturateur (12) sont reliées ensemble par une zone filetée (12c).

22. Ensemble raccord de nébuliseur selon la revendication 19, **caractérisé en ce que** le morceau de tuyau (11) est susceptible d'être obturé par un mécanisme de serrage (19).

23. Ensemble raccord de nébuliseur selon la revendication 22, **caractérisé en ce que** le mécanisme de serrage (19) comprend un élément de serrage (19a) agissant sur un morceau de tuyau.

24. Ensemble raccord de nébuliseur selon la revendication 23, **caractérisé en ce que** l'élément de serrage (19a) est relié à un élément d'actionnement (19b), de manière qu'un mouvement rotatif de l'élément d'actionnement soit transmis à l'élément de serrage.

25. Ensemble raccord de nébuliseur selon la revendication 24, **caractérisé en ce que** l'élément d'actionnement (19b) est conçu de manière que la tubulure de distribution d'aérosol (5a) du nébuliseur (5) agisse sur l'élément d'actionnement, de manière qu'un mouvement de rotation de la tubulure de distribution d'aérosol (5a) soit transmis à l'élément d'actionnement (19b) et, ensuite, à l'élément de serrage (19a).

26. Ensemble raccord de nébuliseur selon l'une des revendications précédentes, **caractérisé en ce qu'**un dispositif de fixation (20) est prévu, au moyen duquel le nébuliseur (5) est maintenu après raccordement.

27. Ensemble raccord de nébuliseur selon la revendication 26, **caractérisé en ce que** le dispositif de maintien comprend des éléments d'encliquetage (21) ou un filetage (22) pour l'interaction avec des éléments d'encliquetage (5d) ou un filetage (5i), prévus sur le nébuliseur (5).

28. Ensemble raccord de nébuliseur selon la revendication 26, **caractérisé en ce que** le dispositif de maintien comprend des saillies (17, 18) pour l'interaction avec des rainures de maintien (5g, 5h), prévues sur le nébuliseur (5).

29. Ensemble raccord de nébuliseur selon la revendication 26, **caractérisé en ce que** le dispositif de maintien est un raccord à baïonnette (30), avec une rainure d'enfichage (31), s'étendant dans la direction longitudinale de la tubulure de distribution (5a), et une rainure de verrouillage (32), s'étendant sur la périphérie de la tubulure de distribution (5a).
